# EUROPEAN PATENT APPLICATION

(11) **EP 1 810 968 A2**
(43) Date of publication of application: **25.07.2007**
(21) Application number: 07008608.7
(22) Date of filing: 27.09.2004
(51) Int. Cl.: C07D 305/14, C07D 493/08, A61K 31/335, A61P 35/00

(54) **9, 10-a, a-OH-texane analogs and methods for production thereof**

(30) Priority: 25.09.2003 US 506680 P
(62) Divisional of application: 04789167.6
(71) Applicant: Tapestry Pharmaceuticals, Inc., Boulder Colorado 80301 (US)
(72) Inventor: McChesney, James D., Boulder CO 80303 (US); Ferrara, James, Boulder CO 80305 (US); Zygmut, Jan, Longmont CO 80503 (US)
(74) Representative: Cole, William Gwyn

(57) **Abstract**

The present invention relates to new taxane analogs useful for the treatment of cancer as well as methods for producing the same. The chemical compounds according to the present invention have the general Formula (I) wherein R₁ and R₂ are each selected from H, an alkyl group, an olefinic group, an aromatic group, an O-alkyl group, an 0-olefinic group, or an O-aromatic group; R₃ is hydroxyl or OP₁; R₄ and R₅ are each hydroxyl or R₇COO; R₆ is hydroxyl, OP₂, R₇COO, or an ether functionally; R₇ is an alkyl group, an olefinic group, or an aromatic group; and P₁ and P₂ are each hydroxyl protecting groups. The chemical compounds of the present invention may particularly be 9, 10-α,α-OH taxane analogs that are formed by a process that begins with a standard taxane as the starting compound.

## Description

### Field of the Invention

The present invention generally relates to chemical compounds for use in treating cancer patients. More particularly, the present invention is directed to new and useful taxane analogs and methods for producing them. Specifically, the present invention relates to 9,10-α,α-OH taxane analogs, production methods and intermediates useful in the formation thereof.

### Background of the Invention

Various taxane compounds are known to exhibit anti-tumor activity. As a result of this activity, taxanes have received increasing attention in the scientific and medical community, and are considered to be an exceptionally promising family of cancer chemotherapeutic agents. For example, various taxanes such as paclitaxel and docetaxel have exhibited promising activity against several different varieties of tumors, and further investigations indicate that such taxanes promise a broad range of potent anti-leukemic and tumor-inhibiting activity.

One approach in developing new anti-cancer drugs is the identification of superior analogs and derivatives of biologically active compounds. Modifications of various portions of a complex molecule may lead to new and better drugs having improved properties such as increased biological activity, effectiveness against cancer cells that have developed multi-drug resistance (MDR), fewer or less serious side effects, improved solubility characteristics, better therapeutic profile and the like.

In view of the promising anti-tumor activity of the taxane family, it is desirable to investigate new and improved taxane analogs and derivatives for use in cancer treatment. One particularly important area is the development of drugs having improved MDR reversal properties. Accordingly, there is a need to provide new taxane compounds having improved biological activity for use in treating cancer. There is also a need to provide methods for forming such compounds. Finally, there is a need for methods of treating patients with such compounds for use in cancer treatment regimens. The present invention is directed to meeting these needs.

### Summary of the Invention

According to the present invention then, new and useful chemical compounds for use in cancer treatment are provided having the formulas: 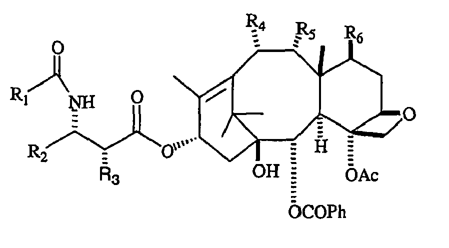 and

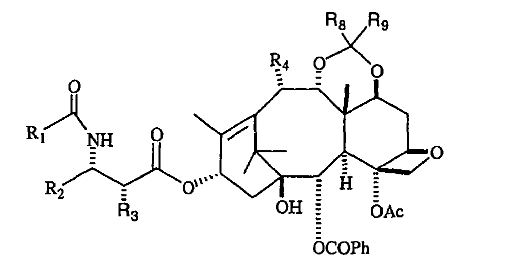

When reference is made to compounds throughout this disclosure, possible R_{X} groups and P_{X} groups contemplated hereby are set forth in the following Table 1:

**TABLE 1**

| R_{X} GROUPS AND P_{X} GROUPS CONTEMPLATED | |
|---|---|
| R₁ | H, an alkyl such as an isobutyl group or a tert-butoxyl group, olefinic such as a tiglyl group, aromatic such as a phenyl group, O-alkyl, O-olefinic, or O-aromatic |
| R₂ | H, alkyl such as an isobutyl group, olefinic, aromatic group such as Ph, O-alkyl, O-olefinic, or O-aromatic |
| R₃ | hydroxyl or OP₁ |
| R₄ | hydroxyl or R₇COO |
| R₅ | hydroxyl or R₇COO |
| R₆ | hydroxyl, OP₂, R₇COO or an ether functionality such as an O-methylthiomethyl or other hetero substituted ethers |
| R₇ | alkyl such as a methyl group, olefinic or aromatic |
| R₇COO | 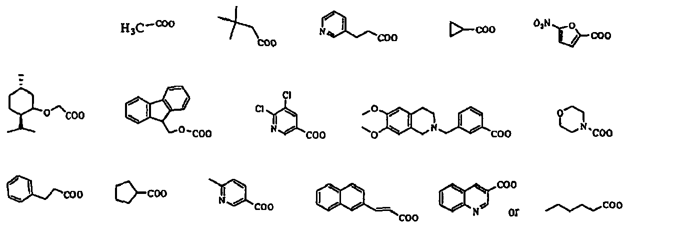 |
| R₈ | H, alkyl group such as a methyl or ethyl group, olefinic or aromatic group |
| R₉ | H, alkyl group such as a methyl or ethyl group, olefinic or aromatic or may specifically be: 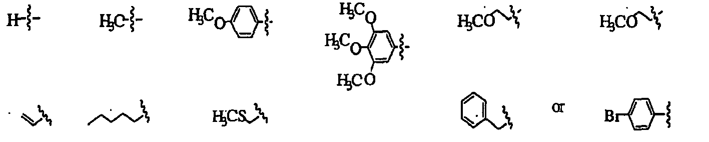 |
| R₁₀ | alkyl group such as a methyl or ethyl group |
| R₁₁ | H, alkyl group such as a methyl or ethyl group, olefinic or aromatic group |
| R₁₂ | H, alkyl group such as a methyl or ethyl group, olefinic or aromatic or may specifically be: 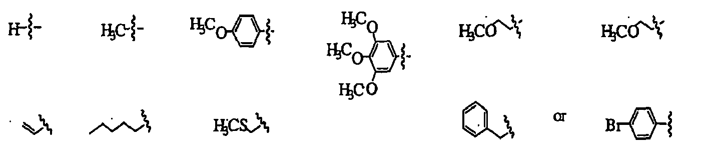 |
| R₁₃ | H, alkyl group such as a methyl or ethyl group, olefinic or aromatic group |
| R₁₄ | H, alkyl group such as a methyl or ethyl group, olefinic or aromatic or may specifically be: 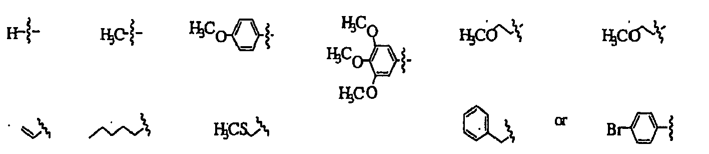 |
| P₁ | hydroxyl protecting group such as a silyl protecting group, for example, TBDMS or TES |
| P₂ | hydroxyl protecting group such as a silyl protecting group, for example, TES |
| P₃ | NH protecting group such as carbobenzyloxy (CBZ) |

Specifically, R₁ may be Ph or tert-butoxyl or tiglyl, R₂ may be Ph or isobutyl, R₆ may be O-methylthiomethyl or other hetero substituted ethers, P₁ may be a silyl protecting group such as TBDMS or TES, and P₂ may be a silyl protecting group such as TES. Compounds according to the present invention may be monoacylated at C-10, such as when R₅ and R₆ are hydroxyl and R₄ is R₇COO, where R₇COO has a formula selected from the following structures: 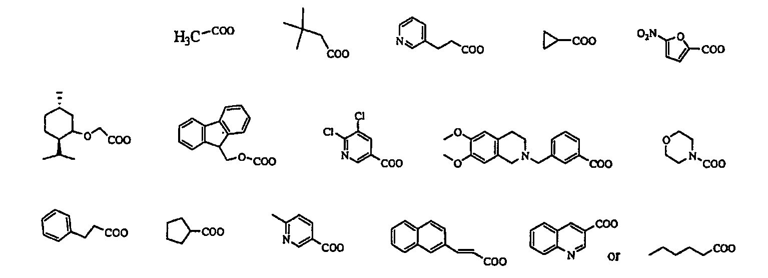 Compounds according to the present invention may alternately be mono-, bis-, or tris-acylated at the 7, 9 and/or 10 positions. For example, R₆ may be R₇COO when R₄ and R₅ are hydroxyl; R₄ and R₆ may both be R₇COO when R₅ is hydroxyl; or each of R₄; R₅ and R₆ may be R₇COO; where R₇COO is:

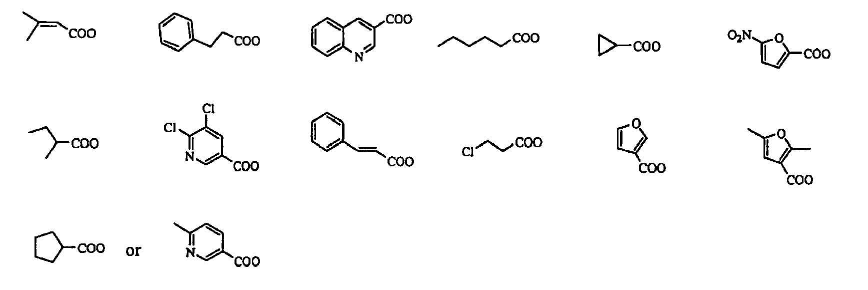

Additionally, chemical compounds according to the present invention may have the formula: 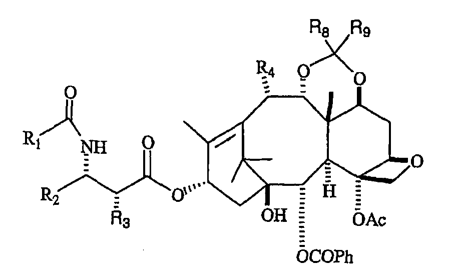 wherein R₁ through R₄ are as defined in Table 1 above and R₈ and R₉ are each H, alkyl, olefinic or aromatic. Compounds according to the present invention may be monoacylated at C10, such as when R₄ is R₇COO, where R₇COO is: 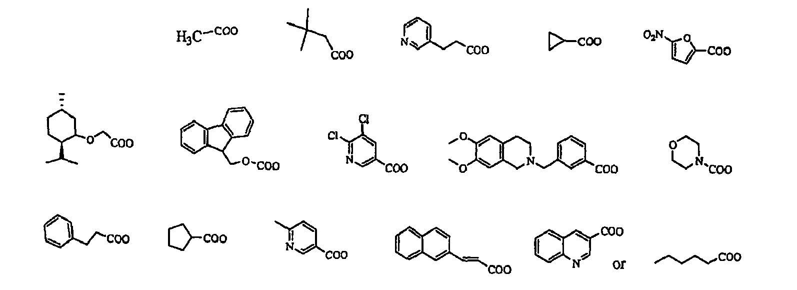 R₈ may specifically be H or methyl, and R₉ may specifically be: 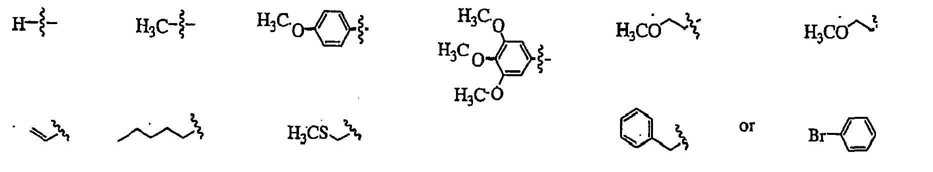 Compounds according to the present invention may have an acroline acetal group connecting the 7,9-positions. For example, chemical compounds of formula: 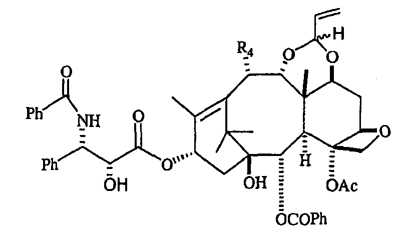 or 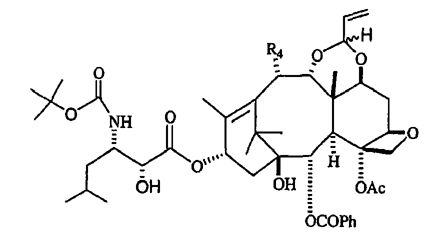 are provided wherein R₄ is hydroxyl or CH₃COO.

Another example of the 7,9-acetal linked compounds contemplated have the formulas:

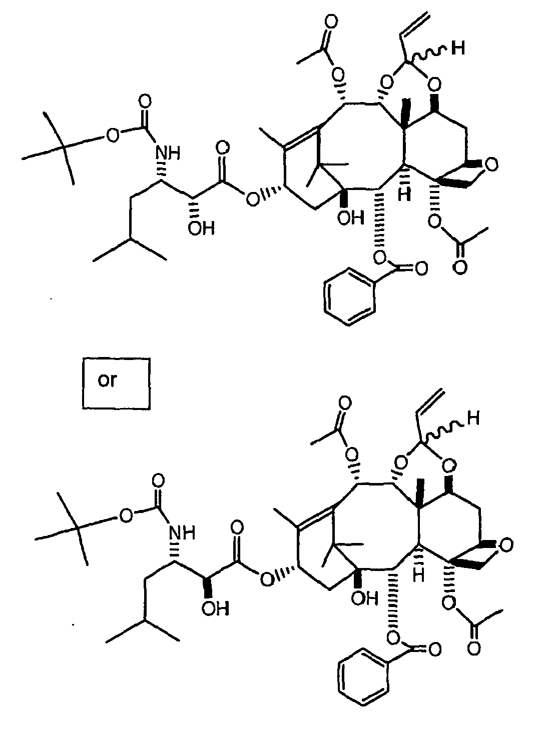

The present invention also provides intermediates for use in forming compounds useful for cancer treatment, comprising: 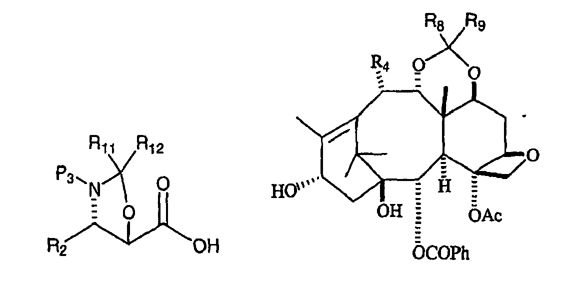 or 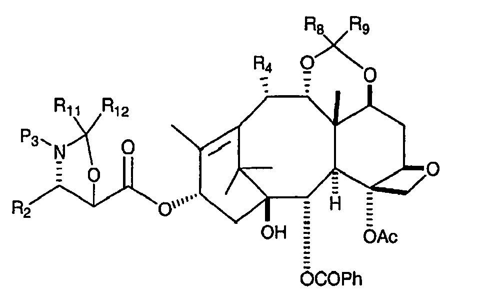 wherein R₂, R₄, R₈ and R₉ are as defined in Table 1 above, P₃ is a NH protecting group such as carbobenzyloxy (CBZ), and R₁₁ and R₁₂ are as defined in Table 1 above for R₈ and R₉, respectively.

The present invention also provides methods for use in producing taxane analogs and derivates thereof for use in cancer treatment. One method according to the present invention comprises providing a starting compound of formula 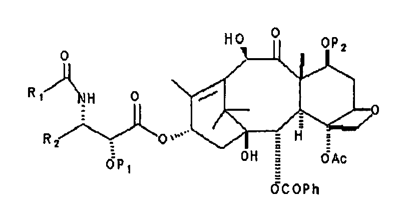 and converting the starting compound into a first taxane analog of the formula 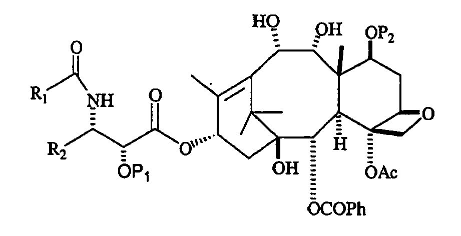 wherein:
R₁ and R₂ are each selected from H, an alkyl group, an olefinic group, an aromatic group, an O-alkyl group, an O-olefinic group, or an O-aromatic group;
R₇ is an alkyl group, an olefinic group, or an aromatic group; and
P₁ and P₂ are each hydroxyl protecting groups;
The starting compound may be oxidized to form a first intermediate compound of the formula 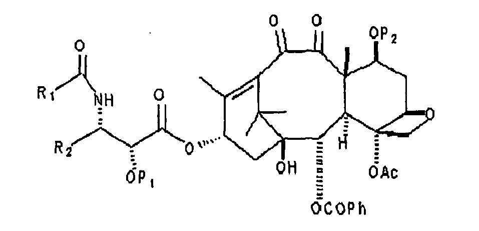 The method may further include the step of acylating the first taxane analog at the C-10 position to form a second taxane analog of formula 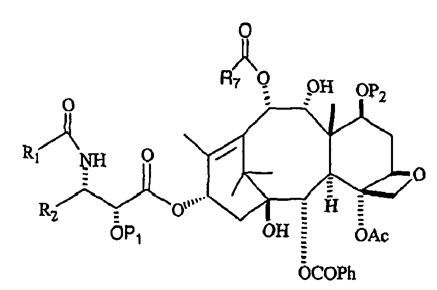 that may thereafter be deprotected, thereby to form a third taxane analog of formula: 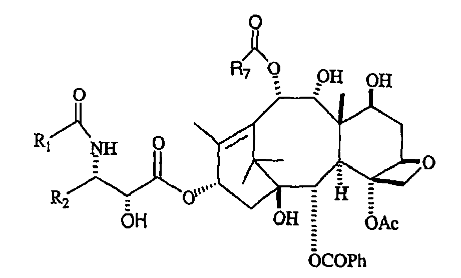 where R₁, R₂, R₇, P₁ and P₂ are as defined in Table 1 above. The acylation step may be accomplished using a carboxylic acid R₇COOH, carboxylic acid halide R₇COX, such as an acid chloride, or a carboxyl anhydride R₇COOCOR₇. When P₁ and P₂ are silyl protecting groups such as TES or TBDMS, the step of deprotecting the second taxane analog may be accomplished in a single step using tetrabutylammoniumfluoride (TBAF). Alternatively, the step of deprotecting the second taxane analog may include a first step of deprotecting the second compound at the C-7 position thereby to form a fourth taxane analog of formula: 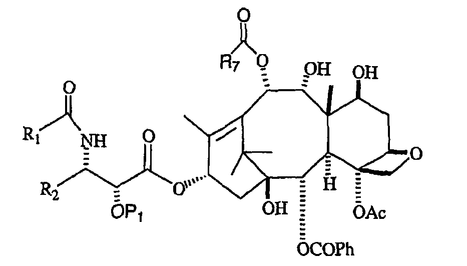 and then deprotecting the 2'O position of the fourth taxane analog to form a fifth taxane analog of formula: 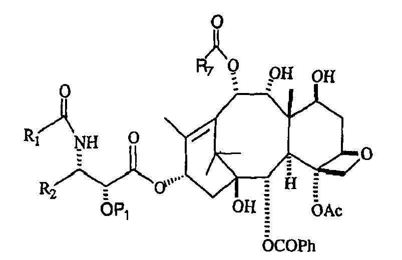 The first step may be accomplished using HF-ACN, and the second step may be accomplished using HF-pyridine.

Alternatively, instead of acylating the first taxane analog, it may be deprotected at the 7-O position to form a sixth taxane analog of formula 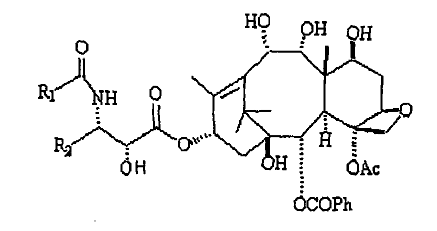 Thereafter the sixth taxane analog may be acylated at the C-7 position, the C-9 position, or the C-10 position to form a seventh taxane analog of formula 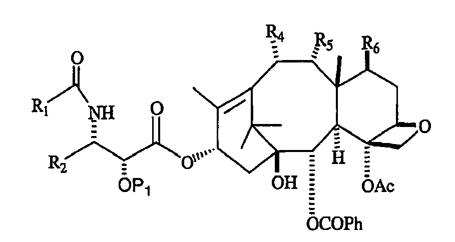 The seventh taxane analog may be deprotected at the 2'O position to form an eight taxane analog of formula 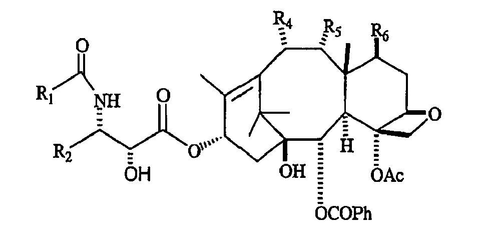 The acylation step of the sixth taxane analog may be accomplished using a carboxylic acid R₇COOH, carboxylic acid halide R₇COX, such as an acid chloride, or a carboxyl anhydride R₇COOCOR₇. Deprotection of the seventh taxane analog at the C-2' position may be accomplished using tetrabutylammoniumfluoride (TBAF).

Another method according to the present invention comprises providing a starting compound of formula 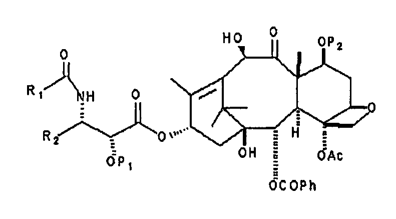 wherein
R₁ and R₂ are each selected from H, an alkyl group, an olefinic group, an aromatic group, an O-alkyl group, an O-olefinic group, or an O-aromatic group;
R₇ is an alkyl group, an olefinic group, or an aromatic group; and
P₁ and P₂ are each hydroxyl protecting groups;
The starting compound may be converted into a first taxane analog of formula 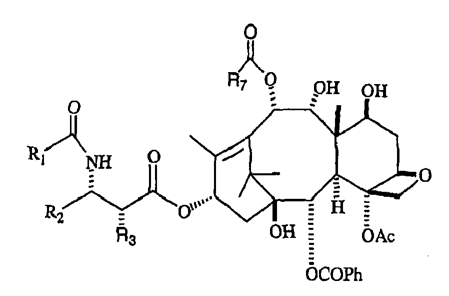 wherein
R₁ and R₂ are each selected from H, an alkyl group, an olefinic group, an aromatic group, an O-alkyl group, an O-olefinic group, and an O-aromatic group;
R₃ is hydroxyl or OP₁;
R₇ = an alkyl group, an olefinic group, or an aromatic group;
P₁ is a hydroxyl protecting group.
The first taxane analog may have a formula selected from the following structures 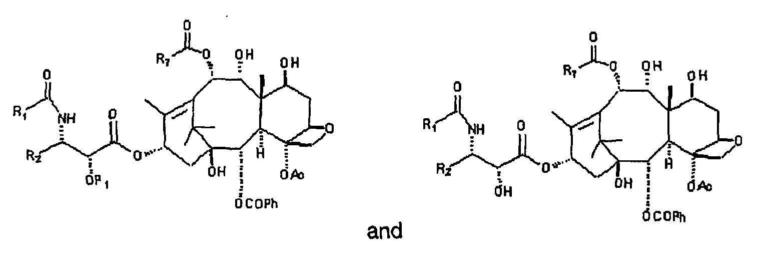 The first taxane analog may then be protected as a 7,9-acetal linked analog to form a second taxane analog of formula 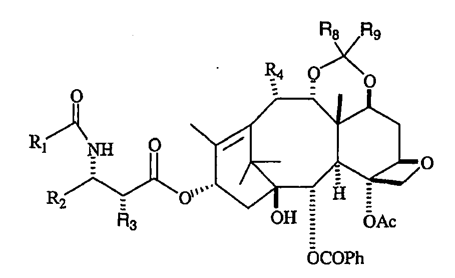 which may specifically have one of the following formulas 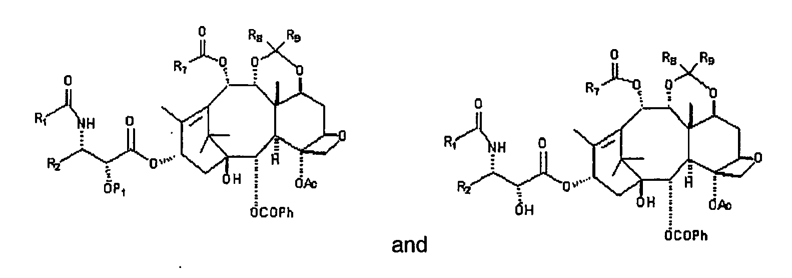 The sidechain of the second taxane analog may thereafter be cleaved at the C-13 position to convert the second taxane analog into a first intermediate compound of formula 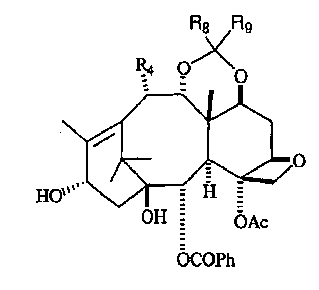 Subsequently, the first intermediate compound may be esterified with a second intermediate compound of formula 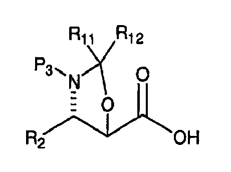 thereby to form a third taxane analog of formula 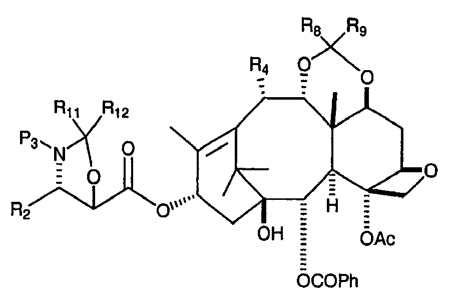 wherein:
R₂ is selected from H, an alkyl group, an olefinic group, an aromatic group, an O-alkyl group, an O-olefinic group, and an O-aromatic group;
R₄ is either hydroxyl or R₇COO;
R₇ is an alkyl group, an olefinic group, or an aromatic group;
R₈, R₉, R₁₁, and R₁₂ are each selected from H, an alkyl group, an olefinic group, or an aromatic group; and
P₃ is NH protecting group.
Specifically, the R9 and R12 moieties may specifically be 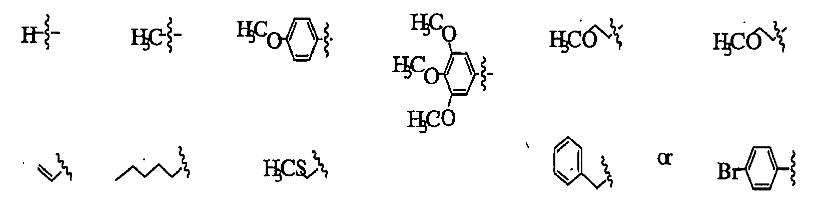 Also, P₃ may specifically be carbonbenzyloxy (CBZ).

A further method according to the present invention comprises converting a first compound of formula: 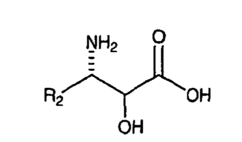 to a second compound of formula: 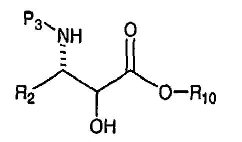 protecting the second compound as an N,O-acetal to form a third compound of formula: 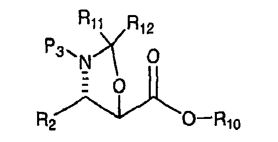 and saponifying the third compound to a fourth compound of formula: 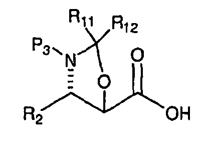 where R₂, R₁₁, R₁₂ and P₃ are as defined in Table 1 above and R₁₀ is an alkyl group such as a methyl or ethyl group.

Finally, the present invention contemplates a method of treating cancer in a patient, comprising administering to the patient a pharmaceutical formulation including a selected concentration of a taxane and a pharmaceutically acceptable carrier therefor, wherein the taxane has a formula: 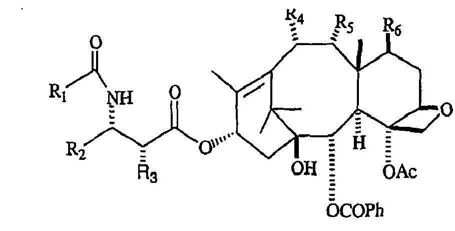 or 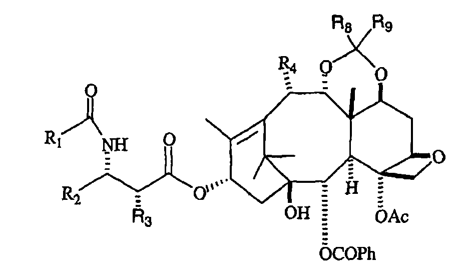 and C-2' S isomers thereof wherein R₁ through R₉ are as defined in Table 1 above.

These and other objects of the present invention will become more readily appreciated and understood from a consideration of the following detailed description of the exemplary embodiments of the present invention when taken together with the accompanying drawings, in which:

### Brief Description of the Drawings

Figure 1 is a diagram of a generalized Scheme 1 for forming 9,10-α,α taxane analogs according to the present invention;
Figure 2 is a diagram of a generalized Scheme 2 for forming 9,10-α,α taxane analogs according to the present invention;
Figure 3 is a diagram of a generalized Scheme 3 for forming 9,10-α,α taxane analogs according to the present invention;
Figure 4 is a diagram of exemplary R₇COO groups for use in Scheme 3;
Figure 5 is a diagram of a generalized Scheme 4 for forming mono-, bis- and tris-acylated 9,10-α,α taxane analogs according to the present invention;
Figure 6 is a diagram of exemplary R₇COO groups for use in Scheme 4;
Figure 7 is a diagram of a generalized Scheme 5 for forming 9,10-α,α taxane analogs according to the present invention;
Figure 8 is a diagram of a generalized Scheme 6 for forming 9,10-α,α-7,9-acetal taxane analogs according to the present invention;
Figure 9 is a diagram of exemplary compounds formed according to Scheme 6;
Figure 10 is a diagram of a generalized Scheme 7 for cleaving the sidechain of 9,10-α,α taxane analogs according to the present invention;
Figure 11 is a diagram of a generalized Scheme 8 for forming a carboxylic acid for use in attaching an alternative taxane sidechain to 9,10-α,α taxane analogs according to the present invention;
Figure 12 is a diagram of a generalized Scheme 9 for esterifying the sidechain of Figure 11 to a 13-hydroxy-9,10-α,α taxane analog according to the present invention;
Figure 13 is a diagram of an exemplary 2'-hydroxyl protection of paclitaxel according to the present invention;
Figure 14 is a diagram of an exemplary 10-deacylation of the compound formed in Figure 13;
Figure 15 is a diagram of an exemplary 7-hydroxyl protection of the compound formed in Figure 14;
Figure 16 is a diagram of an exemplary 10-hydroxyl oxidation of the compound formed in Figure 15;
Figure 17 is a diagram of an exemplary 9,10-diketo reduction of the compound formed in Figure 16;
Figure 18 is a diagram of an exemplary 10-acylation of the compound formed in Figure 17;
Figure 19 is a diagram of exemplary 7-deprotections of the compounds formed in Figures 17 and 18;
Figure 20 is a diagram of exemplary 2'-deprotections of the compounds formed in Figure 19;
Figure 21 is a diagram of exemplary 2',7-deprotections of the compounds formed in Figures 17 and 18;
Figure 22 is a diagram of exemplary mono-, bis- and tris-acylations of a compound formed in Figure 19, where R₇COO may be selected from the formulas of Figure 6;
Figure 23 is a diagram of an exemplary 2'-protection of a compound formed in Figures 20 and 21;
Figure 24 is a diagram of an exemplary 7-O-methylthiomethylation of the compound formed in Figure 23;
Figure 25 is a diagram of an exemplary 2-deprotection of the compound formed in Figure 24;
Figure 26 is a diagram of an exemplary 7,9-acetalization reaction of a compound formed in Figure 19;
Figure 27 is a diagram of an exemplary 2'-deprotection of the compound formed in Figure 26;
Figure 28 is a diagram of an exemplary 7,9-acetalization of a compound formed in Figures 20 and 21;
Figure 29 is a diagram of an exemplary reaction for cleaving the taxane sidechain of the compound formed in Figure 28;
Figure 30 is a diagram of an exemplary reaction for producing an isobutyl N-protected ester compound for use in forming an alternative taxane sidechain according to the present invention;
Figure 31 is a diagram of an exemplary reaction for protecting the compound formed in Figure 30 as an anisaldehyde acetal;
Figure 32 is a diagram of an exemplary reaction for saponifying the compound formed in Figure 31 to a carboxylic acid;
Figure 33 is a diagram of an exemplary reaction for attaching the sidechain compound formed in Figure 32 to the 13-hydroxy taxane analog formed in Figure 29;
Figure 34 is a diagram of an exemplary deprotection and acylation of the compound formed in Figure 33; and
Figure 35 is a diagram of an exemplary 7,9-acetalization of the compound formed in Figure 34.

### Detailed Description of the Invention

Paclitaxel and docetaxel have a formula as follows: 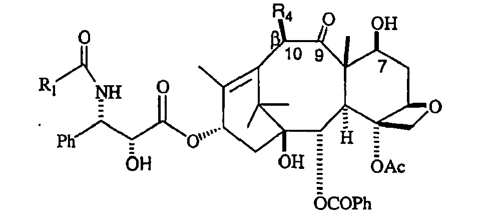 **Paclitaxel:** R₁ = Ph, R₄ = AcO
**Docetaxel** R₁= t-Butoxy, R₄ = OH

Of note is the top part of the molecule as illustrated above, which may be seen to have a 9-keto structure and 10-β hydroxy or 10-β acetyl stereochemistry. The present invention provides novel taxane analogs having α stereochemistry at the C-9 and C-10 OH positions of the molecule. Generally, these compounds have been found to exhibit excellent inhibition of cell growth against MDR sensitive cancer cell lines. For example, the 9,10-α,α hydroxy taxane derivatives discussed in Table 2 exhibit favorable inhibition of cell growth in several of the tested cell lines.

**TABLE 2**

| **BIOLOGICAL ACTIVITY DATA OF SELECTED TAXANES** | | | | | |
|---|---|---|---|---|---|
| **Cancer Type & Cell line** | **MDR** | **Tubulin** | **Agent** | **Concentration** | **Inhibition** |
| Ovarian Carcinoma 1A9PTX10 | + | Mutant | Paclitaxel | 5 ug/mL | 55% |
| Ovarian Carcinoma 1A9PTX10 | + | Mutant | TPI 287 | 0.2 ug/mL | 85% |
| Ovarian Carcinoma 1A9PTX10 | + | Mutant | TPI287 | 0.1 ug/mL | 51% |
| Ovarian Carcinoma 1A9PTX10 | + | Mutant | TPI 251 | 0.5 ug/mL | 96% |
| Ovarian Carcinoma 1A9PTX10 | + | Mutant | TPI 251 | 0.25 ug/mL | 93% |
| Breast Cancer MCF-7 NCI-AR | + | Wild Type | Paclitaxel | 40 ug/mL | 55% |
| Breast Cancer MCF-7 NCI-AR | + | Wild Type | TPI 287 | 0.5 ug/mL | 80% |
| Breast Cancer MCF-7 NCI-AR | + | Wild Type | TPI 287 | 0.25 ug/mL | 47% |
| Breast Cancer MCF-7 NCI-AR | + | Wild Type | TPI 287 | 0.125 ug/mL | 37% |
| Breast Cancer MCF-7 NCI-AR | + | Wild Type | TPI 287 | 0.061 ug/mL | 22% |
| Breast Cancer MCF-7 NCI-AR | + | Wild Type | TPI 287 | 0.031 ug/mL | 13% |
| Breast Cancer MCF-7 NCI-AR | + | Wild Type | TPI 251 | 2.0 ug/mL | 94% |
| Breast Cancer MCF-7 NCI-AR | + | Wild Type | TPI 251 | 1.0 ug/mL | 65% |
| Breast Cancer MCF-7 NCI-AR | + | Wild Type | TPI 251 | 0.5ug/mL | 45% |
| Breast Cancer MCF-7 NCI-AR | + | Wild Type | TPI 285 | 2.0 ug/mL | 85% |
| Breast Cancer MCF-7 NCI-AR | + | Wild Type | TPI 285 | 1.0 ug/mL | 51% |
| Breast Cancer MCF-7 NCI-AR | + | Wild Type | TPI 285 | 0.5 ug/mL | 41% |
| Neuroblastoma SK-N-AS | - | Wild Type | Paclitaxel | 0.1 ug/mL | 54% |
| Neuroblastoma SK-N-AS | - | Wild Type | TPI 287 | 0.05 ug/mL | 58% |
| Squamous Cell Carcinoma FADU | - | Wild Type | Paclitaxel | 0.05 ug/mL | 47% |
| Squamous Cell Carcinoma FADU | - | Wild Type | TPI 287 | 0.05 ug/mL | 56% |

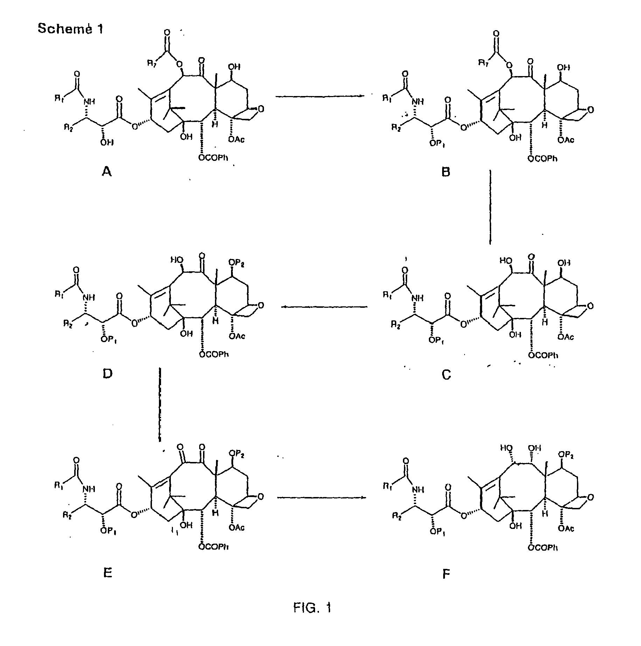

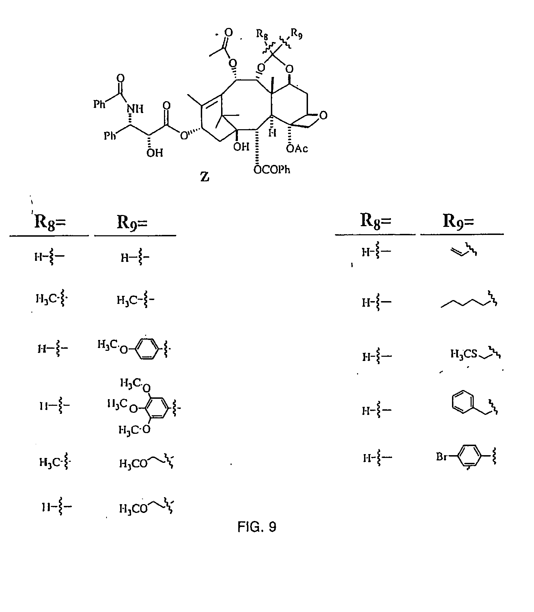

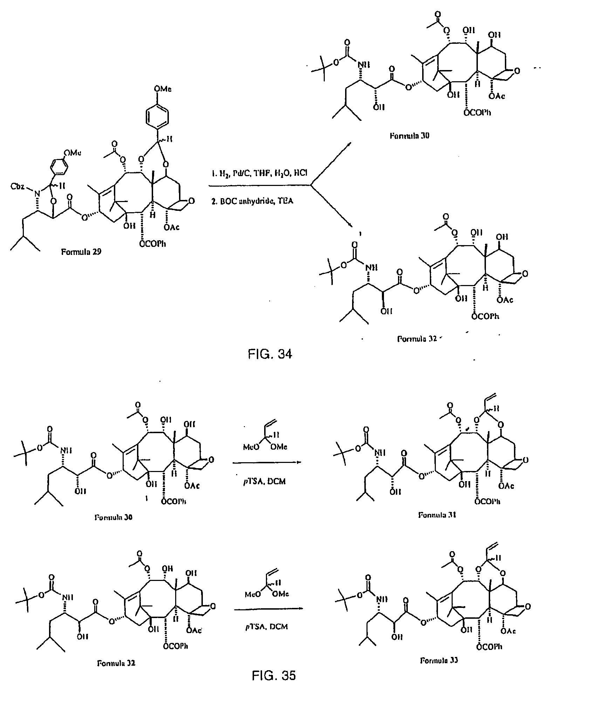

Table 2, above, identifies the compounds TPI 287, TPI 285 and TPI 251, which were found to exhibit excellent inhibition of cell growth against MDR sensitive cancer cell lines. The compounds TPI 287, TPI 285 and TPI 251 are discussed in greater detail below and have the following respective structures: 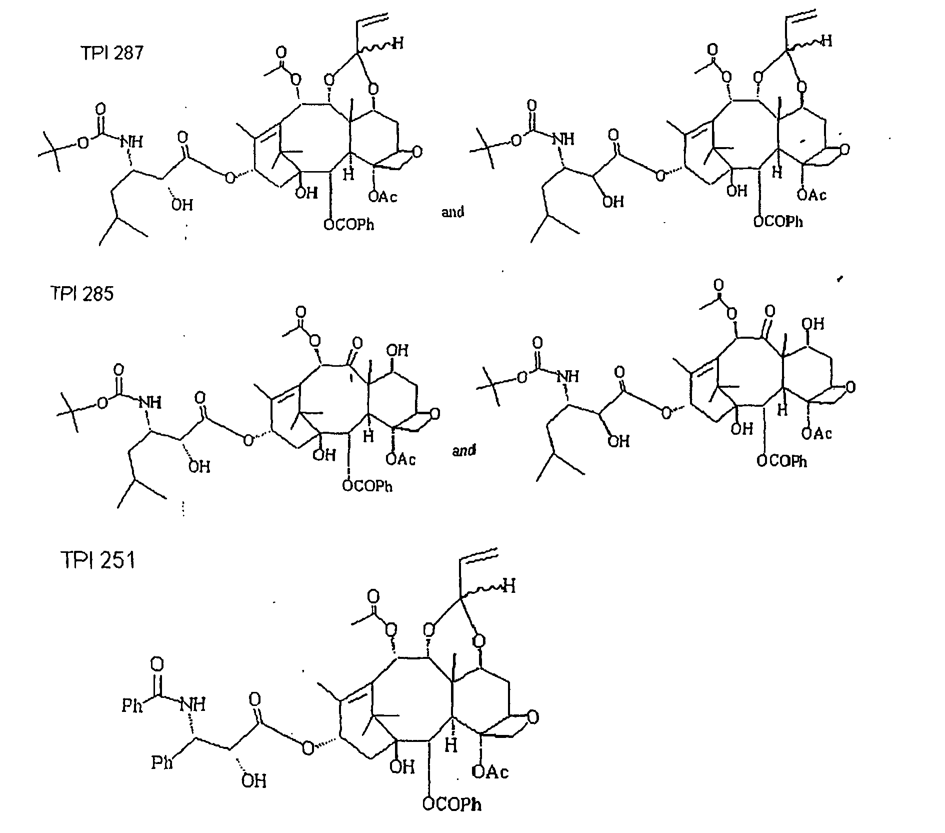 As will become apparent from the discussion below, TPI 287 is a mixture of the compounds identified as Formula **31** and Formula **33,** which are discussed below with respect to Figure 35. The 2'R isomer of TPI 285 is illustrated, for example, with respect to generalized Formula **A** in Figure 1 wherein R₁ is a tort-butoxyl group, R₂ is an isobutyl group and R₇ is acetyl. Although not shown in Figure 1, the 2'S isomer of TPI 285, as shown above, is also contemplated. TPI 251 is illustrated, for example, with respect to generalized Formula **Z** in Figure 9 wherein R₈ is H and R₉ is ethylene. In addition to the compounds TPI 287, TPI 285, and TPI 251, various other 9,10-α,α hydroxy taxane derivatives have also exhibited significant inhibition against various cancer cell lines.

### I. Synthesis of 9,10-α,α-hydroxy taxanes

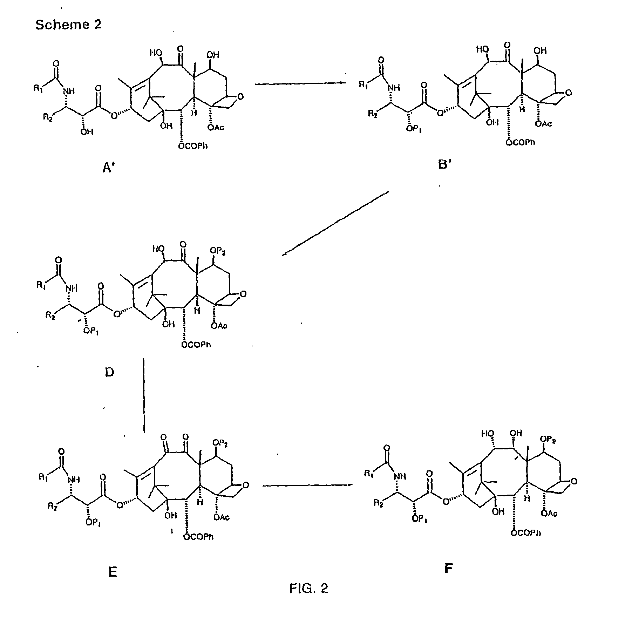

Such compounds may be formed in a number of ways according to the present invention. For example, as shown in Figure 1 (Scheme 1) and Figure 2 (Scheme 2), a 9,10-α,α hydroxy taxane **F** may be formed directly from a standard taxane **A** or **A'** through various transformations, including oxidation of a 10-hydroxy taxane **D** to a 9,10-diketo taxane **E** and reduction to the 9,10-α,α-hydroxy taxane **F.** In the compounds shown in Schemes 1 and 2, R₁ and R₂ may each be H, alkyl such as an isobutyl group or a tert-butyl group, olefinic such as a tigloyl group, aromatic such as a phenyl group, O-alkyl, O-olefinic, or O-aromatic; R₇ may be alkyl such as a methyl group, olefinic or aromatic; and P₁ and P₂ may each be a hydroxyl protecting group, such as a silyl protecting group, including TBDMS or TES.

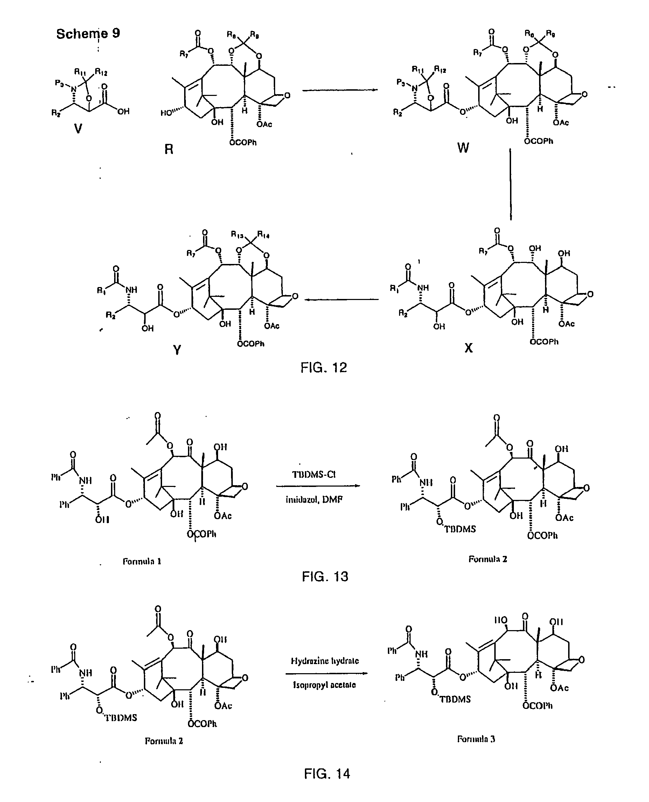

Such a process is exemplified in Figures 13 through 17. For example, as shown in Figure 13, paclitaxel of Formula **1** (where R₁=R₂=Ph; R₇=CH₃ in generalized formula **A** of Scheme 1) is first protected at the 2'-hydroxyl with a hydroxyl protecting group such as *tert*-butyldimethylsilyl (TBDMS). To a 500mL round bottom flask (RBF) equipped with a magnetic stir bar was charged 50.0g (58.55mmol) paclitaxel, Formula **1,** 13.96g (204.8mmol, 3.5eq.) imidazole, and 26.47g (175.7mmol, 3.0eq.) TBDMS-Cl. The flask was placed under a nitrogen environment and 350mL (7mL/g paclitaxel) anhydrous N,N-dimethyl formamide (DMF) was charged to the flask. The reaction was stirred at room temperature for twenty hours, then was worked up by diluting the reaction solution in 600mL isopropyl acetate (IPAc) and washing with water until the aqueous wash reached pH 7, then with brine. The organic partition was dried over magnesium sulfate, filtered and then was evaporated to a white foam solid to yield 66.9g (93.0 area percent) of unpurified 2'-O-TBDMS paclitaxel product of Formula **2** (where R₁=R₂=Ph; R₇=CH₃; P₁=TBDMS in generalized formula **B** of Scheme 1). This reaction is nearly quantitative. There are slight amounts of 2',7-bis-TBDMS, but this is not a significant amount.

Next, as shown in Figure 14, the 10-acetyl group is removed by hydrazinolysis. To a 1 L RBF equipped with a magnetic stir bar was charged 59.5g 2'-O-TBDMS paclitaxel of Formula **2** and 600mL (10mL/g) IPAc. The solution was stirred to dissolve the 2'-O-TBDMS paclitaxel, then 60mL (1 mUg) hydrazine hydrate was charged to the flask and the reaction stirred at room temperature for one hour. The reaction was worked up by diluting the reaction solution in 1.2L IPAc and washing first with water, then ammonium chloride solution, then again with water until the aqueous wash was pH 7 and lastly with brine. The organic partition was dried over magnesium sulfate, filtered and evaporated to 55.8g of solid. The solid was redissolved in 3:1 IPAc (1%water):heptane to a concentration 0.25g/mL total dissolved solids (TDS) and purified on a YMC silica column; the column eluent was monitored for UV absorbance. The fractions were pooled based on HPLC analysis and evaporated to yield 39.3g (98.6 area percent) of 2'-O-TBDMS-10-deacetyl paclitaxel solid of Formula **3** (where R₁=R₂=Ph; P₁=TBDMS in generalized formula C of Scheme 1). If the reaction goes too long (beyond 2h), the product begins epimerizing at the C-7 position. Besides decreasing the yield by the formation of the 7-epi degradant, this impurity requires adding a chromatographic step to remove the impurity.

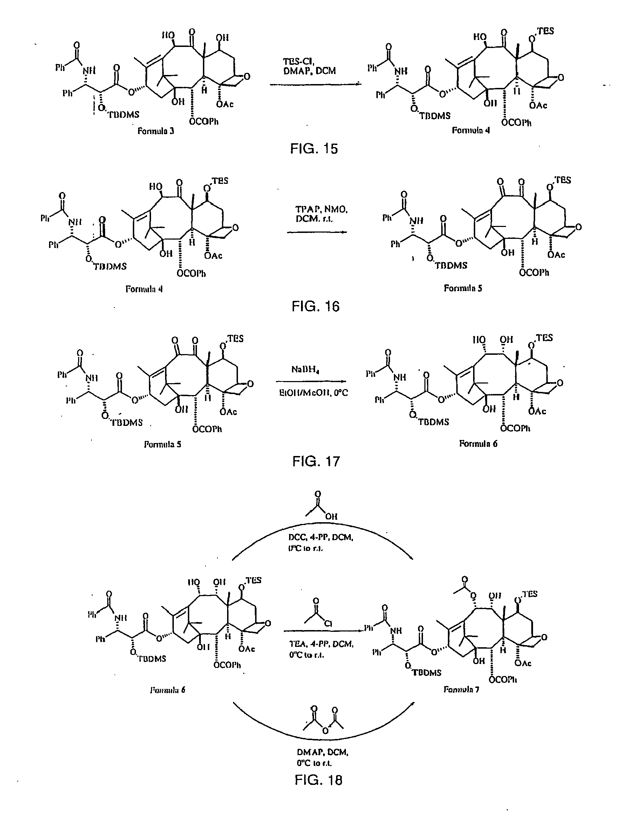

As illustrated in Figure 15, the 7-hydroxyl is now protected with a protecting group such as triethylsilyl (TES). To a 500mL RBF equipped with a magnetic stir bar was charged 39.3g (42.46mmol) 2'-O-TBDMS-10-deacetyl paclitaxel of Formula 3 and 15.6g (127.4mmol, 3eq.) DMAP. The flask was placed under nitrogen and 390mL (10mL/g) anhydrous dichloromethane (DCM) charged to the flask to dissolve the solids followed by 14mL (84.92mmol, 2eq.) TES-Cl. The reaction was stirred at room temperature for three hours. The reaction was worked up by evaporating the reaction solution to approximately half its starting volume and diluting it in 300mL EtOAc and washing with water and dilute HCl solutions until the pH of the aqueous wash was approximately 7, then with brine. The organic partition was dried over magnesium sulfate and evaporated to yield 42.0g (97.7 area percent) of white solid of Formula **4** (where R₁=R₂=Ph; P₁= TBDMS; P₂=TES in generalized formula **D** of Scheme 1). This reaction is nearly quantitative, with a slight amount of 7,10-bis-TES and excess silyl compounds in the worked up solids, as with the 2'-TBDMS protection step above.

Next, oxidation of the 10-hydroxyl yields a 9,10-diketo compound, as exemplified in Figure 16. To a 1 L RBF equipped with a magnetic stir bar was charged 41.0g (39.43mmol) 2'-O-TBDMS-7-O-TES-10-deacetyl paclitaxel of Formula **4,** 2.1g (5.92mmol, 0.15eq.) of TPAP, 13.9g (118.3mmol, 3eq.) NMO. The flask was placed under nitrogen and 720mL (~20mL/g) anhydrous DCM charged to the flask to dissolve the solids. The reaction was stirred at room temperature for 22hours. The reaction was worked up by concentrating the reaction solution to half its volume and then drying the reaction contents onto 175g silica gel (EM Sciences 40-63µ). The taxane containing silica was placed on 30g of clean silica gel (EM Sciences 40-63µ) and the product eluted from the silica with 4L MTBE. The MTBE was evaporated to yield 37.3g (93.2 area percent) 2'-O-TBDMS-7-O-TES-9,10-diketo paclitaxel of Formula **5** (where R₁=R₂=Ph; P₁=TBDMS; P₂=TES in generalized formula **E** of Scheme 1).

Finally, reduction of the 9,10-diketo taxane yields the 9,10-α,α-hydroxy taxane, as shown for example in Figure 17. To a 2L RBF equipped with a magnetic stir bar was charged 37.3g (35.9mmol) protected 9,10-diketo paclitaxel of Formula 5 and 900mL (~30mL/g taxane) of 3:1 EtOH/MeOH. The solution was stirred to dissolve the solids then the flask was placed in an ice/water bath and the solution was stirred for 30 minutes. 8.1g (215.7mmol, 6eq.) of sodium borohydride (NaBH₄) was charged to the flask and the reaction stirred in the ice/water bath for five hours. The reaction was worked up by diluting the reaction solution in 1 L IPAc and washing with 4 x 750mL water, then with 200mL brine. The organic partition was dried over magnesium sulfate. The aqueous washes were reextracted with 500mL IPAc. The organic reextract solution was washed with 100mL brine then dried over magnesium sulfate and combined with the first organic partition. The IPAc solution was concentrated until solids began precipitating out then heptane was added to the solution to crystallize the protected 9,10-α,α-OH, 9-desoxo, 10-deacetyl paclitaxel product of Formula 6 (where R₁=R₂=Ph; P₁=TBDMS; P₂=TES in generalized formula **F** of Scheme 1). The crystallizing solution was placed in a freezer overnight. Three crystallizations were done on the material, the first yielded 4.1g (95.3 area percent) protected 9,10-α,α-OH, 9-desoxo, 10-deacetyl paclitaxel product, the second yielded 18.3g (90.9 area percent) product, and the third yielded 2.9g (81.7 area percent) product. The original work on this reaction employed flash chromatography to purify the product. However, the crystallizations that were performed gave similar purity, by HPLC, to the chromatographed material from earlier work.

As illustrated in Figure 2 (Scheme 2), the same steps as above may be followed-absent the hydrazinolysis step-when the starting material is a 10-deacetyl taxane, such as of generalized Formula **A'** in Figure 2.

### II. 10-Acylation and 2',7-Deprotection

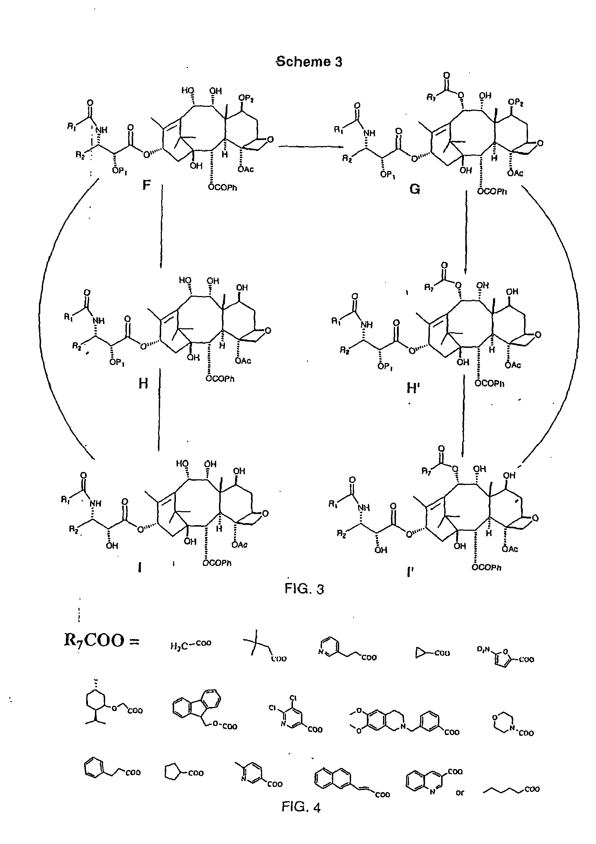

Next, as shown in Figure 3 (Scheme 3), the resulting taxane of generalized formula **F** may be deprotected at the 7- position to yield the taxane of generalized formula **H** and then deprotected at the 2'-position to yield a taxane of the generalized formula **I.** The deprotection at the 2'- and 7-positions may be either a two-step process or may be performed in a single step.

Alternatively, as shown in Scheme 3, the taxane of generalized formula **F** may be first acylated at the 10 position before deprotecting at the 7 and 2' positions. According to this route, the 10 acylation of the taxane of generalized formula **F** results in the taxane of generalized formula **G,** which may then be deprotected at 7 position to yield a taxane of the generalized formula **H'** and deprotected at the 2'-position to yield a taxane of the generalized formula **I'**. Here again, the deprotection at the 7- and 2'-positions may be either a two-step process or may be performed in a single step.

The 10-acylation of the taxane of generalized formula **F** may be accomplished in a number of manners, as exemplified in Figure 18. In particular, the invention contemplates the use of either a carboxylic acid of generalized formula R₇COOH, a carboxylic acid halide such as an acid chloride of generalized formula R₇COCl, or a carboxyl anhydride of generalized formula R₇COOCOR₇. In the compounds shown in Scheme 3, R₁, R₂, R₇, P₁ and P₂ are as defined above for Schemes 1 and 2, although it should be appreciated that the R₇COO group attached at C-10 in Scheme 3 may be different from the R₇COO group that was removed in Scheme 1.

When the reagent used is a carboxylic acid, an exemplary procedure (as shown in Figure 18) is as follows. To a 25mL RBF equipped with a magnetic stir bar was charged 300mg (0.288mmol) of 2'-O-TBDMS-7-O-TES-9,10-α,α-OH, 9-desoxo, 10 deacetyl paclitaxel of Formula 6 (where R₁=R₂=Ph; P₁= TBDMS; P₂=TES in generalized formula F of Scheme 3), (0.720mmol, 2.5eq.) carboxylic acid (CH₃COOH), 178mg (0.864mmol, 3.0eq.) of DCC, and 13mg (0.086mmol, 0.3eq.) of 4-pyrrolidinopyridine (4-Pp). The contents of the flask were placed in a nitrogen environment and 10mL anhydrous DCM added to the flask. The reactions were stirred at room temperature for 15+ hours (all the reactions were monitored by TLC or HPLC for consumption of the starting material); the reactions generally ran overnight. The reactions were worked up by diluting the reaction solution in 20mL EtOAc and stirring for fifteen minutes to precipitate dicyclohexyl urea (DCU). The DCU was removed from the solution by vacuum filtration and the filtrate was washed with water until the pH of the water washes was approximately 7. The organic solution was then washed with brine and dried over sodium sulfate before evaporating to dryness.

When the reagent used is a carboxylic acid halide, an exemplary procedure (as shown in Figure 18) is as follows. To a 25mL RBF, equipped with a magnetic stir bar and under a nitrogen environment, was charged 300mg (0.288mmol) of 2'-O-TBDMS-7-O-TES-9,10-α,α-OH, 9-desoxo 10 deacetyl paclitaxel of Formula **6,** (0.720mmol, 2.5eq.) acid chloride (CH₃COCl), 140µL (1.008mmol, 3.5eq.) TEA, 13mg (0.086mmol, 0.3eq.) 4-Pp, and 10mL anhydrous DCM. The reactions were stirred at room temperature for 15+ hours; reactions generally ran overnight and were monitored by TLC and/or HPLC in the morning for consumption of starting material. The reactions were worked up by diluting the reaction solution in 20mL EtOAc and washing with water until the pH of the water washes was approximately 7. The organic solution was then washed with brine and dried over sodium sulfate before evaporating to dryness.

When the reagent used is a carboxyl anhydride, an exemplary procedure (as shown in Figure 18) is as follows. To a 25mL RBF, equipped with a magnetic stir bar and under a nitrogen environment, was charged 300mg (0.288mmol) 2'-O-TBDMS-7-O-TES-9,10-α,α-OH, 9-desoxo 10 deacetyl paclitaxel of Formula 6, (2.880mmol, 10eq.) acid anhydride (CH₃COOCOCH₃), 106mg (0.864mmol, 3eq.) DMAP, and 5mL anhydrous DCM. The reactions were stirred at room temperature for 15+ hours. The reactions were worked up by adding 5mL saturated sodium bicarbonate solution to the reaction flask and stirring for 5 minutes. The solution was then transferred to a seperatory funnel and organics were extracted with 20mL EtOAc. The organic extract was then washed with saturated sodium bicarbonate and water until the pH of the water washes was approximately 7. The organic partition was then washed with brine and dried over sodium sulfate before evaporating to dryness.

The resulting product is the 2'-O-TBDMS-7-O-TES-9-α-OH,9-desoxo,10-*epi* paclitaxel of Formula 7 (where R₁=R₂=Ph; P₁= TBDMS; P₂=TES; R₇=CH₃ in generalized formula **G** of Scheme 3). Figure 4 shows numerous alternative groups that may be used for the R₇COO group at the 10-α-position of generalized formula **G.** As would be appreciated by the ordinarily skilled person, these acylations may be performed for example by substituting the appropriate carboxylic acid R₇COOH, carboxylic acid halide R₇COX or carboxyl anhydride R₇COOCOR₇ in the above procedures.

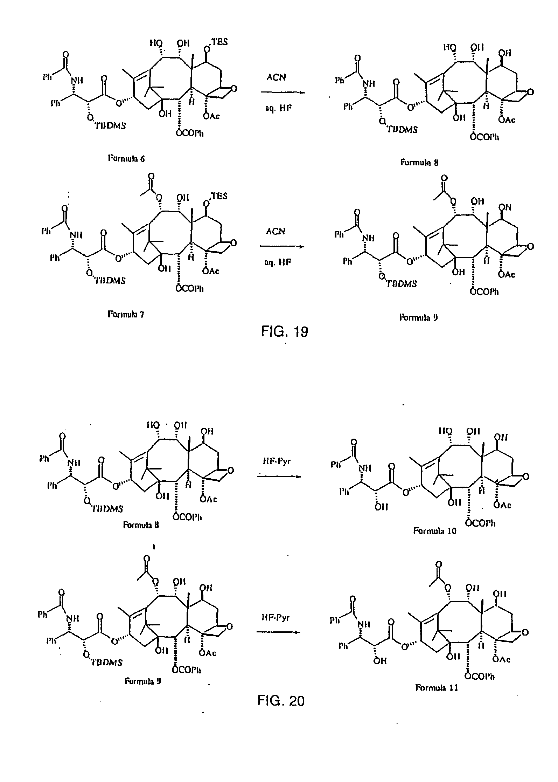

As indicated above and as further illustrated in Scheme 3, taxanes of generalized formula **F** or **G** may be deprotected at the 2'- and 7-positions in either a two-step process or a single step. Figures 19 through 21 show exemplary deprotections of the 2'- and 7-positions.

For example, as shown in Figure 19, the 7-O-TES group may be removed from Formula **6** to give Formula **8** (where R₁=R₂=Ph; P₁=TBDMS in generalized formula **H** of Scheme 3) or from Formula **7** to give Formula **9** (where R₁=R₂=Ph; P₁= TBDMS; R₇=CH₃ in generalized formula **H'** of Scheme 3), respectively, using acetonitrile (ACN) and aqueous HF. To a 500mL teflon bottle equipped with a magnetic stir bar was charged 2.50g (2.40mmol) 2'-O-TBDMS-7-O-TES-9,10-α,α-OH, 9-desoxo, 10deacetyl paclitaxel of Formula **6** and 100mL ACN. The bottle was placed in and ice/water bath and the solution was stirred for 30 minutes. Next, 0.8mL of 48% HF aqueous was added slowly to the reaction solution and the reaction stirred in the ice/water bath for 20 minutes. The reaction was monitored by TLC for disappearance of the starting material. The reaction was worked up by diluting the reaction solution by adding 200mL EtOAc and quenching the acid by adding 25mL saturated sodium bicarbonate solution to the bottle and stirring for 10 minutes. The solution was then transferred to a separatory funnel and the organic partition was washed with water until the pH of the water wash was approximately 7, then was washed with brine. The organic partition was dried over sodium sulfate and then was evaporated to a solid of Formula **8.** This procedure was also followed if there was an acyl group on the 10-α-hydroxyl (i.e. Formula **7** to Formula **9** in Figure 19 or generalized formula **G** to generalized Formula **H'** in Scheme 3).

Next, as shown in Figure 20, the 2'-O-protecting group may be removed from Formula **8** to give Formula **10** (where R₁=R₂=Pₕ in generalized formula **I** of Scheme 3) or from Formula **9** to give Formula **11** (where R₁=R₂=Ph; R₇=CH₃ in generalized formula **I'** of Scheme 3), respectively. To a 50mL teflon bottle equipped with a magnetic stir bar was charged, 500mg 2'-O-TBDMS-9,10-α,α-OH, 9-desoxo, 10-deacetyl paclitaxel of Formula **8** (or 2'-O-TBDMS-9-α-OH, 9-desoxo,10-*epi* paclitaxel of Formula **9)** and 5mL anhydrous THF. Next, 1 mL HF-pyridine solution was slowly charged to the reaction solution. The reaction was stirred at room temperature for 1 hour; reaction progress was monitored by TLC and/or HPLC for disappearance of starting material. The reaction was worked up by adding 10mL EtOAc to the bottle to dilute the reaction solution then saturated sodium bicarbonate was slowly added to the bottle to neutralize the HF. The solution was then transferred to a separatory funnel and the organic partition was washed with 10 wt% sodium bicarbonate solution then water until the pH of the water wash was approximately 7. Then the organic partition was washed with brine and then dried over sodium sulfate before evaporating to a solid of Formula **10** (or Formula **11).**

It should be appreciated that one ordinarily skilled in the art would understand that the order of the above deprotection steps may be reversed, such that the 2'-hydroxyl protecting group is removed first, and the 7-hydroxyl protecting group removed second.

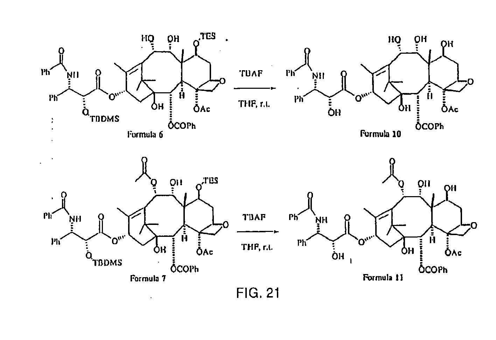

Further, as indicated above, the 2'- and 7-positions of either the taxanes of the generalized formula **F** or **G** may be deprotected in a one-step procedure using tetrabutylammoniumfluoride (TBAF). Here, as shown for example in Figure 21, Formula **6** may be deprotected directly to Formula **10,** and Formula **7** may be deprotected directly to Formula 11. A 10mL RBF equipped with a magnetic stir bar was charged with 100mg of 2'-O-TBDMS-7-O-TES-9,10-α,α-OH, 9-desoxo 10 deacetyl paclitaxel of Formula **6** (or 2'-O-TBDMS-7-O-TES-9-α-OH-10-*epi* paclitaxel of Formula 7) and 5mL EtOAc or THF to dissolve the taxane. Next, 100µL of. 1M TBAF in THF was charged to the flask and the reaction was stirred at room temperature for 1 hour; the reaction was monitored by TLC and/or HPLC for disappearance of starting material. The reaction was worked up by washing the reaction solution with water and then brine. The organic partition was dried over sodium sulfate and evaporated to a solid of Formula **10** (or Formula **11).** This method removes both the 2'-O-TBDMS protecting group and the 7-O-TES protecting group.

### III. 7,9,10-Acylation

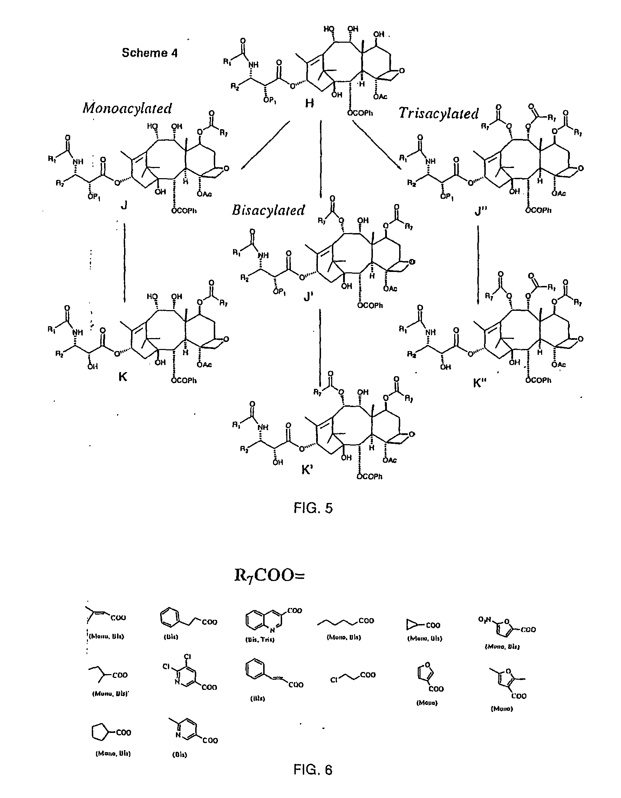

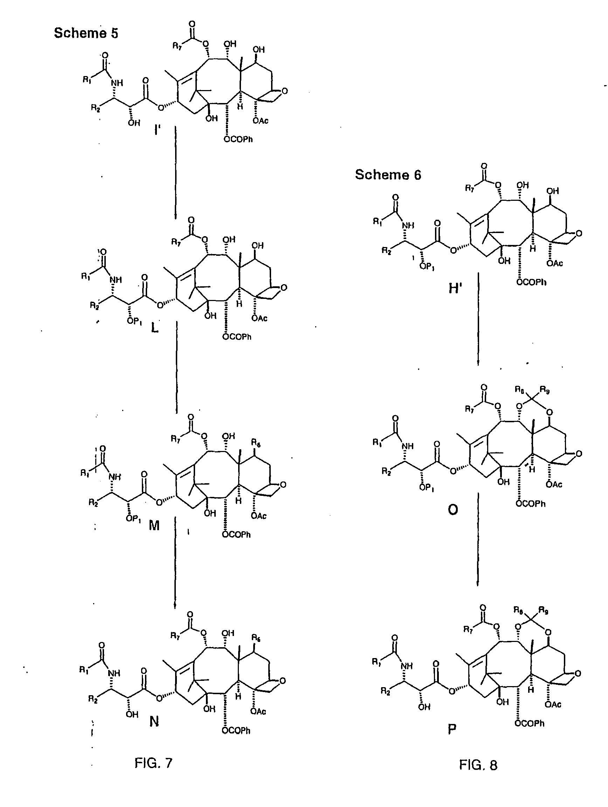

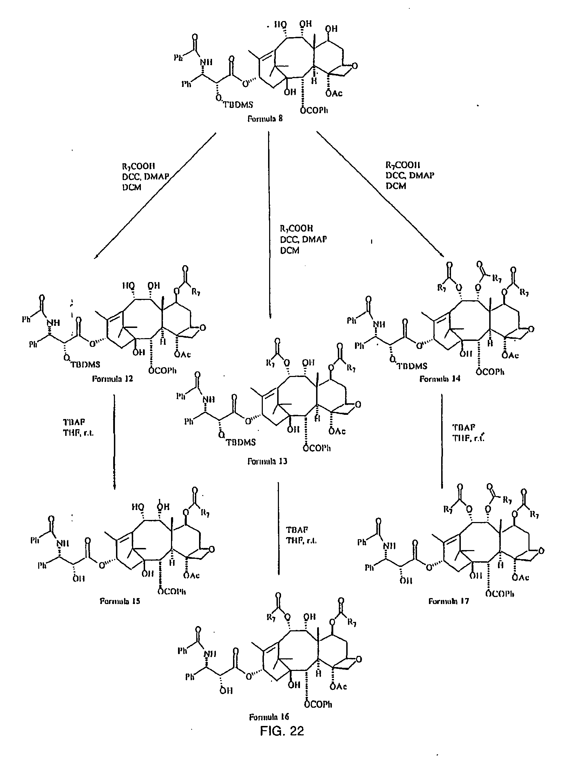

Now, as illustrated in Figure 5 (Scheme 4), the 7-, 9-, and/or 10-positions may be acylated with various groups R₇COO, such as those shown in Figure 6. In the compounds shown in Scheme 4, R₁, R₂, R₇, and P₁ are as defined above for Schemes 1 and 2, although it should be appreciated that the R₇COO groups in Scheme 4 may be different from the R₇COO group that was removed in Scheme 1. For example, as shown in Figure 22, 2'-O-TBDMS-9,10-α,α-OH, 9 desoxo, 10 deacetyl paclitaxel of Formula **8** (where R₁=R₂=Ph; P₁= TBDMS in generalized formula **H** of Scheme 4) may be mono-acylated on the 7-hydroxyl as Formula **12** (corresponding to generalized formula **J** of Scheme 4), bis-acylated on the 7,10-hydroxyls as Formula **13** (corresponding to generalized formula **J'** of Scheme 4), and/or tris-acylated on the 7,9,10-hydroxyls as Formula **14** (corresponding to generalized formula **J"** of Scheme 4). It should be appreciated by the ordinarily skilled person that the appropriate carboxylic acid R₇COOH corresponding to the desired R₇COO group may be substituted in the procedure below, such as those groups from Figure 6 or other groups as desired. To a 5mL RBF, equipped with a magnetic stir bar and nitrogen purge, was charged 100mg (0.108mmol) 2'-O-TBDMS-9,10-α,α-OH, 9 desoxo, 10 deacetyl paclitaxel of Formula **8,** (0.324mmol, 3eq.) carboxylic acid, 66.8mg (0.324mmol, 3eq.) DCC, 6.6mg (0.054mmol, 0.5eq.) DMAP, and 1.5mL anhydrous DCM. The reaction was stirred at room temperature for 2.5 hours. The reaction progression was monitored by TLC and/or HPLC. If no acyl addition was detected, an additional charge of reagents was done to try and start the reaction. The reaction produces a mixture of monoacylated, bisacylated, and some trisacylated products. The reaction was worked up by filtering the reaction solution through a 0.2µm nylon acrodisc. To the filtrate plus a 1 mL DCM wash of the solids 100mg of IRC-50 ion-exchange resin was added. The mixture was stirred at room temperature for 30 minutes. The mixture was filtered again through a second 0.2µm nylon acrodisc. As further shown in Figure 22, the resulting filtrate solution went directly to the reaction to remove the TBDMS from the 2'-hydroxyl using the TBAF method, described above to obtain formula **10** and formula **11** from formula **6** and formula **7** respectively; 150µL of the reagent was added directly to the filtrate and stirred at room temperature for four hours. The work-up was the same as described above for the deprotection method. Compounds were purified on a reverse phase semi-prep scale HPLC column to provide Formula **15** (corresponding to generalized formula **K** of Scheme 4), Formula **16** (corresponding to generalized formula **K'** of Scheme 4) and Formula **17** (corresponding to generalized formula **K"** of Scheme 4).

### IV. 7-Ether Functionality

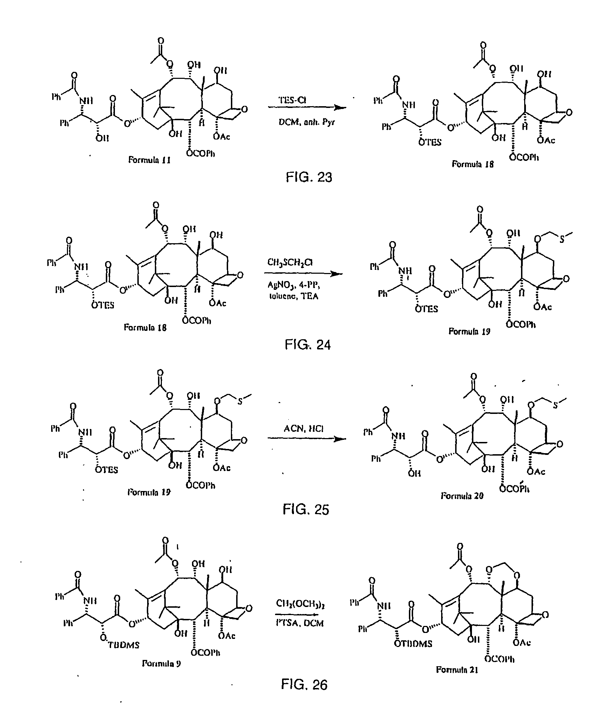

As illustrated in Figure 7 (Scheme 5) the 2'-hydroxyl may be protected and a functional group attached at the C-7 position, as shown for example in Figures 23 through 25. In the compounds shown in Scheme 5, R₁, R₂, R₇, and P₁ are as defined above for Scheme 3, and R₆ is an ether functionality, such as an O-methylthiomethyl group or other hetero substituted ether functionalities. Initial attempts to synthesize a 7-O-methylthiomethyl compound from 2'-O-TBDMS-9-α-OH-10-*epi* paclitaxel provided difficulty in that the methylthiomethyl group was too labile to withstand the 2'-hydroxyl deprotection step using either the HF-pyridine method or the TBAF method, described above. Accordingly, it is desirable to use a 2'-hydroxyl protecting group that can be removed under less harsh conditions, such as a TES protecting group. In Figure 23, 9-α-OH-10-*epi* paclitaxel of Formula **11,** which may be formed according to one of the routes described above with respect to Scheme 3, is first protected as the 2'-O-TES ether of Formula **18** (where R₁=R₂=Ph; P₁=TES; R₇=CH₃ in generalized formula **L** of Scheme 5). To a 25mL RBF, equipped with a magnetic stir bar and a nitrogen purge, was charged 1.2g (1.415mmol) 9-α-OH-10-*epi* paclitaxel of Formula **11,** 6mL anhydrous DCM, and 6mL anhydrous pyridine. The flask was placed in an ice/water bath and the solution was stirred for 15 minutes. After the solution cooled, 0.95mL (5.659mmol, 4.0eq.) TES-Cl was charged to the flask. The reaction was stirred in the ice/water bath for 3 hours. The reaction was worked up by diluting the reaction solution in 30mL EtOAc and washing with water then brine. The organic partition was dried over sodium sulfate before evaporating to a solid. The 2'-O-TES-9-a-OH-10-epi paclitaxel product of Formula **18** was purified by flash chromatography using an EtOAc/heptane gradient.

As shown for example in Figure 24, a methylthiomethyl group may be attached at the 7-O-position to give Formula **19** (where R₁=R₂=Ph; P₁=TES; R₇=CH₃; R₆=OCH₂SCH₃ in generalized formula **M** of Scheme 5). Because the C-9 hydroxyl is very susceptible to oxidation, it is preferred that there are no oxidizing reagents present in the reaction to add the methylthiomethyl ether to the modified taxane. A 100mL RBF was equipped with a magnetic stir bar, a nitrogen purge, and a condenser, and was wrapped with aluminum foil. 850mg (0.877mmol) 2'-O-TES-9-α-OH-10-*epi* paclitaxel of Formula **18,** 894mg (5.261 mmol, 6eq.) silver nitrate, 156mg (1.052mmol, 1.2 eq.) 4-Pp, 50mL anhydrous toluene, and 0.8mL (5.701mmol, 6.5eq.) TEA were charged to the flask. The solution was stirred to dissolve the solids then 441µL (5.261mmol, 6.0eq.) chloromethylmethyl-sulfide was charged to the flask. The reaction was heated to 70 °C. The reaction was stirred at 70 °C for 24 hours. The reaction was worked up by filtering the reaction solution through Celite. The reaction flask and solids were washed with 80mL EtOAc. The combined filtrate was transferred to a separatory funnel and washed with water then dilute ammonium chloride then dilute sodium bicarbonate then with water until the pH of the water wash was approximately 7. Next the organic partition was washed with brine then dried over sodium sulfate before it was concentrated to approximately 5mL. This solution was purified by flash chromatography using an EtOAc/heptane gradient. The fraction pools were evaporated to yield 0.13g of 2'-O-TES-7-O-methylthiomethyl-9-α-OH-10-*epi* paclitaxel of Formula **19**.

The 2'-hydroxyl is then deprotected, as shown for example in Figure 25 to provide Formula 20 (where R₁=R₂=Ph; R₇=CH₃; R₆=OCH₂SCH₃ in generalized formula N of Scheme 5). To a 10ml RBF, equipped with a magnetic stir bar, 0.12g (0.117mmol) 2'-O-TES-7-O-methylthiomethyl-9-α-OH-10-*epi* paclitaxel of Formula **19** and 8mL ACN were charged. The flask was placed in an ice/water bath and the solution stirred for 30 minutes. 233µL (0.233mmol, 2eq.) of 1 N HCl was charged to the flask and the reaction stirred in the ice/water bath for 45 minutes. The methylthiomethyl ether is fairly acid labile, and the methylthiomethyl group may be removed if the reaction to remove the TES group using 1 N HCl in ACN runs too long. The reaction was worked up by pouring the reaction solution into a separatory funnel containing 20mL EtOAc and 30mL saturated sodium bicarbonate solution. After agitation the aqueous partition was removed and the organic partition was washed with water until the pH of the water wash was approximately 7 then with brine. The organic partition was dried over sodium sulfate then evaporated to a yellowish oil. The product was purified by reverse phase semi-prep scale HPLC to yield 50mg of 7-O-methylthiomethyl-9-α-OH-10-*epi* paclitaxel of Formula **20** as a white solid.

### V. 7,9-Acetal Linked Analogs

As illustrated in Figure 8 (Scheme 6) the present invention also provides 7,9 acetal linked analogs of 9,10-α,α OH taxanes. In particular, the 7- and 9-positions may be linked through a generalized -OC(R₈)(R₉)O- structure and the 2'-position may be deprotected. In the compounds shown in Scheme 6, R₁, R₂, R₇ and P₁ are as defined above for Scheme 3, and R₈ and R₉ may each be H, alkyl, olefinic or aromatic. Figure 9 illustrates various 7,9-acetal linked analogs of formula **Z** formed according to the method described below. Initial data from a cytotoxicity study on the compound where R₈=R₉=H in Figure 9 suggested that there was good activity for the acetal. It should be appreciated that the present invention contemplates further variations in the substituents of such 7,9-acetal linked analogs. For example, the R₈ and R₉ groups shown in Figure 9, or others, may be substituted for R₈ and R₉ in the generalized formulas **O** and **P** of Scheme 6, and the R₁, R₂, R₇ and P₁ groups thereof may be further varied as described herein.

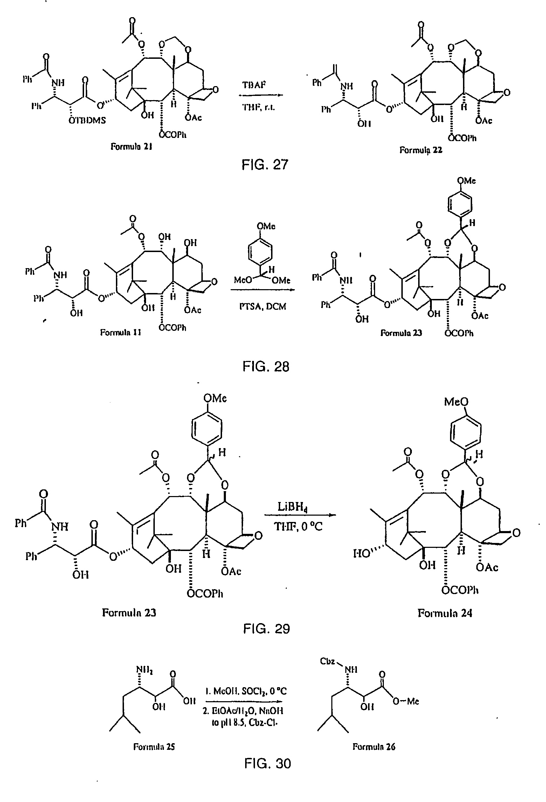

For example, as shown in Figure 26, a compound of Formula **9** (which may be formed as described above with respect to Figure 19) may be protected as a 7,9-acetal linked analog of Formula **21** (where R₁=R₂=Ph; P₁= TBDMS; R₇=CH₃; R₈=R₉=H in generalized formula **O** of Scheme 6). To a 10mL RBF, equipped with a magnetic stir bar and nitrogen purge, 100mg (0.103mmol) 2'-O-TBDMS-9-α-OH-10-*epi* paclitaxel of Formula **9,** 2.5mg (0.013mmol, 0.13eq.) p-toluene sulfonic acid, and 5mL anhydrous DCM were added. The solution was stirred to dissolve the solids then CH₂(OCH₃)₂ (0.515mmol, 5eq.) was added and the reaction was stirred at room temperature for 1.5 hours. Reaction progress was monitored by TLC and/or HPLC. The reaction was worked up by diluting the reaction solution in 10mL and washing the resulting solution with water then brine. The organic partition was dried over sodium sulfate and evaporated to a solid of Formula **21**. The protected product was purified on a reverse phase semi-prep scale HPLC before running the TBAF deprotection method, as shown in Figure 27, to remove the TBDMS group to form Formula **22** (where R₁=R₂=Ph; R₇=CH₃; R₈=R₉=H in generalized formula **O** of Scheme 6). As apparent from Scheme 6, it should be appreciated that compounds of generalized formula R₈R₉C(OCH₃)₂ may be substituted in the reaction above to provide 7,9-acetal linked analogs having R₈ and R₉ groups, such as those illustrated in Figure 9 or others.

### VI. Replacement of Taxane Sidechain

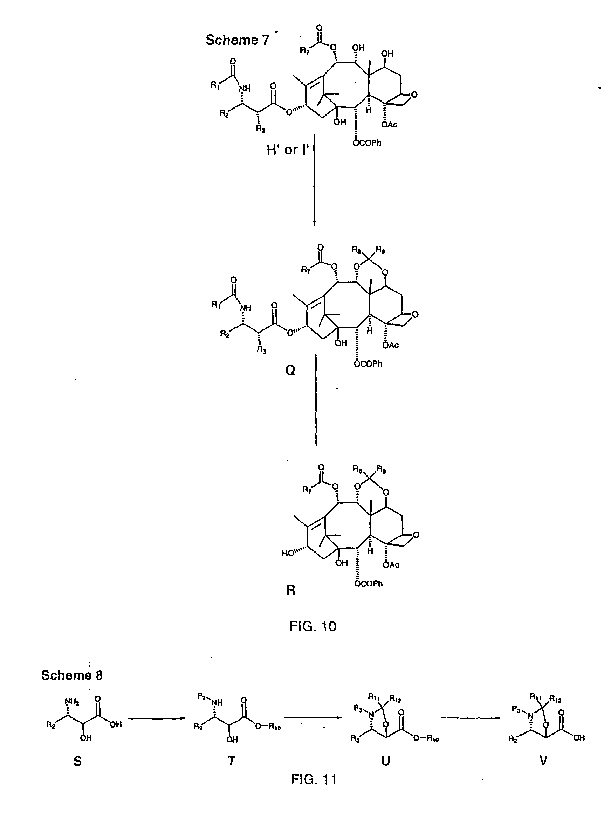

The discussion above and the corresponding figures illustrate various methods of producing 9,10-α,α-OH taxanes as well as intermediate compounds useful in the formation of those taxanes. With respect to those 9,10-α,α-OH taxanes produced by those methods, the sidechain may be cleaved therefrom so as to attach an alternative sidechain having different substituents than those shown and described. Accordingly, Figure 10 provides a generalized Scheme 7 for cleaving the sidechain of 9,10-α,α-OH taxane analogs according to the present invention. The sidechain may be replaced, for example, with a compound of Formula 12 according to the generalized Scheme 9, shown in Figure 12.

More particularly, as shown in Scheme 7 and exemplified in Figures 28 and 29, a 9,10-α,α-taxane may be protected as a 7,9-acetal linked analog, such as described above, and the sidechain may thereafter be cleaved to provide a 13-hydroxyl taxane. In the compounds shown in Scheme 7, R₃ is hydroxyl or OP₁; R₁, R₂, R₇ and P₁ are as defined above for Scheme 3; and R₈ and R₉ are as defined above for Scheme 6.

For example, a compound of Formula 11 was first prepared according to procedures described above with respect to Figures 18 and 21, as follows. To a 200mL RBF was charged 5.0g (4.800mmol) 2'-O-TBDMS-7-O-TES-9,10-α,α-OH, 9 desoxo 10 deacetyl paclitaxel (Formula **6),** 1.75g (14.400mmol, 3.0 eq.) DMAP, and 60mL anhyd. DCM to dissolve the solids. The flask was sealed and placed under nitrogen then the flask was placed in an ice-water bath. Next was slowly charged, 4.5mL (48.000mmol, 10.0 eq.) acetic anhydride to the flask. The reaction was stirred at 0 °C, going to room temperature overnight. The reaction was quenched after 18 hours by adding 100mL of saturated sodium bicarbonate solution. Product was extracted with EtOAc and washed with sodium bicarbonate solution and with water. The organic partition was dried down to yield approximately 5.5g (5.075mmol) of crude product of Formula **7.** This crude product was charged in a 250mL RBF with 110mL THF, under nitrogen. Next was charged 14.2mL of 1.0M TBAF in THF. The reaction was stirred at room temperature for 2.5 hours then was worked up by extracting with EtOAc and washing with water. The organic partition was evaporated to yield approximately 5.9g of crude solid. The crude material was purified by flash chromatography to yield 1.5g of purified compound of Formula **11.**

As shown for example in Figure 28, the compound of Formula **11** may be protected as a 7,9-acetal such as with anisaldehyde dimethyl acetal to form a compound of Formula **23** (where R₁=R₂=Ph; R₃=OH; R₇=CH₃; R₈=H; R₉=PhOMe in generalized formula **Q** of Scheme 7). To a 50mL RBF was charged 1.15g (1.345mmol) 9-α-OH-10-*epi* paclitaxel of Formula **11** and 25mL anhydrous DCM, under nitrogen. 343µL (2.017mmol, 1.5 eq.) anisaldehyde dimethyl acetal was charged to the flask, followed by 51 mg (0.269mmol, 0.2 eq.) PTSA. The reaction was stirred at room temperature for 45 minutes then was worked up by extracting the product with EtOAc and washing with saturated sodium bicarbonate solution followed by water. The organic partition was evaporated to yield approximately 1.5g of crude product. The crude product was purified by flash chromatography to yield 0.72g of pure product of Formula **23.**

Next, the sidechain is cleaved to form the compound of Formula **24** (where R₇=CH₃; R₈=H; R₉=PhOMe in generalized formula **R** of Scheme 7), as exemplified in Figure 29. To a 25mL RBF was charged 720mg (0.740mmol) 7,9-anisaldehyde acetal-10-*epi* paclitaxel of Formula **23** and 15mL anhyd. THF, under nitrogen. The flask was placed in an ice/water/ammonium chloride, -13 °C bath. Solid lithium borohydride (29.0mg, 1.331 mmol, 1.8 eq.) was charged to the reaction flask and the reaction stirred at -13°C for two hours before raising the temperature to 0 °C. The reaction was worked up after five hours fifteen minutes by diluting with EtOAc and washing with water and ammonium chloride solution. The organic partition was evaporated to yield 650mg of crude compound but HPLC indicated that there was only approximately 20% product and mostly unreacted starting material; therefore, the reaction was restarted by repeating the above procedure and running the reaction for an additional six hours. The organic partition was evaporated to yield approximately 660mg of crude product. The compound was purified on a YMC silica column to yield the compound of Formula **24.**

The replacement sidechain may next be formed as illustrated in Figure 11 (Scheme 8) and shown in Figures 30 through 32, for example. In the compounds shown in Scheme 8, R₂ is as defined above for Schemes 1 and 2; P₃ is a hydroxyl protecting group such as a carbobenzyloxy (CBZ) group; R₁₀ is an alkyl group such as a methyl or ethyl group; and R₁₁ and R₁₂ are defined as for R₈ and R₉, respectively, for Scheme 6 above. It should be appreciated that the R₂ group attached at C-3 in Scheme 8 may be different from the R₂ group that was on the sidechain that was removed in Scheme 7. Further, while the exemplary diagrams show an isobutyl sidechain, it should be appreciated that other groups may be substituted for the various substituents in the formulas of Scheme 8.

As shown in Figure 30, a carboxylic acid of Formula **25** (where R₂=CH₂CH(CH₃)₂ in generalized formula S of Scheme 8) is converted to an ester of Formula **26** (where R₂=CH₂CH(CH₃)₂; P₃=CBZ; R₁₀=methyl in generalized formula **T** of Scheme 8). To a 1 L RBF was charged 8.65g (53.69mmol) 2-R,S-hydroxy-3-S-amino-5-methyl hexanoic acid of Formula **25,** and 130mL MeOH to suspend the acid. The flask was then placed in an ice-water bath and 17.6mL (241.62mmol, 4.5 eq.) thionyl chloride (SOCl₂) was slowly charged to the flask. The reaction was stirred at 0 °C for four and a half hours then 160mL EtOAc and 100mL water was charged to the flask and the pH of the reaction solution was adjusted to approximately 8 using 3M NaOH. Next, 16.9mL (118.1mmol, 2.2 eq.) CBZ-CI was charged to the flask and the pH was then readjusted to approximately 8. The reaction was stirred an additional three hours before working it up by diluting the reaction with EtOAc, removing the aqueous partition and washing the organic solution with water before evaporating it to yield approximately 22g of crude oil. The product was purified by normal phase chromatography to yield 8.4g of product of Formula **26.**

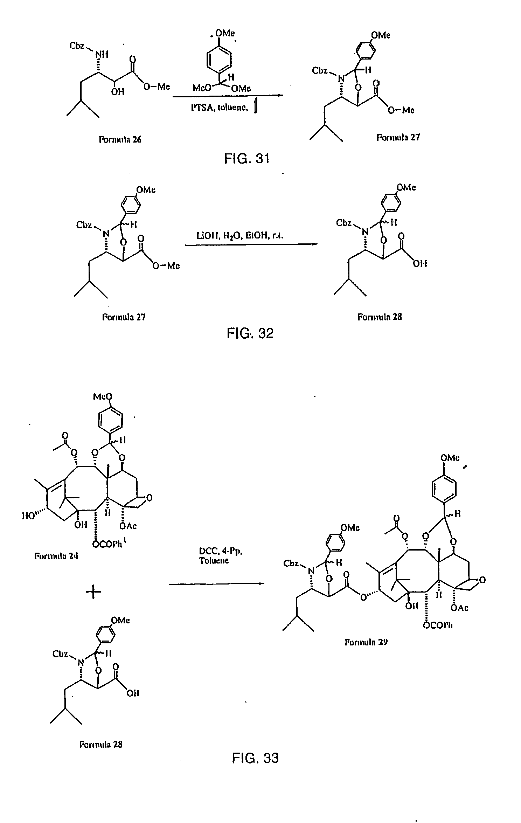

As shown in Figure 31, the compound of Formula **26** may be protected as an N,O-anisaldehyde acetal of Formula **27** (where R₂=CH₂CH(CH₃)₂; P₃= CBZ ; R₁₀=methyl; R₁₁=H; R₁₂=PhOMe in generalized formula U of Scheme 8). To a 10mL RBF equipped with a reflux condenser was charged 250mg (0.809mmol) 2-R,S-hydroxy-3-S-N-(Cbz)-5-methyl hexanoyl methyl ester and 6mL toluene to dissolve the solid. Next was charged 15mg (0.081mmol, 0.1 eq.) PTSA followed by 165µL (0.970mmol, 1.2 eq.) anisaldehyde dimethyl acetal. The reaction was refluxed for two and a half hours then was quenched by washing the reaction solution with 4mL of saturated sodium bicarbonate solution. The organic partition was evaporated to an oil then was purified by flash chromatography to yield 218mg of product of Formula **27.**

While it is preferred that the N,O-acetal protecting the sidechain is the same as the 7,9-acetal protecting the taxane backbone (i.e. R₈=R₁₁ and R₉=R₁₂) so that they may both be removed later in a single chemical step, it should be appreciated that different acetal protecting groups may be used, and separate deprotection steps may be necessary.

As shown in Figure **32,** the ester compound of Formula **27** is next saponified to its corresponding carboxylic acid of Formula **28** (where R₂=CH₂CH(CH₃)₂; P₃=CBZ; R₁₁=H; R₁₂=PhOMe in generalized formula **V** of Scheme 8). To a 5mL RBF was charged 280mg (0.656mmol) of 3-N,2-O-anisaldehyde acetal-3-N-Cbz-5-methyl hexanoyl methyl ester of Formula **27** and 2.8mL EtOH to dissolve the solid. Next was charged a solution of 51.3mg LiOH monohydrate in 420µL water. The reaction was stirred at room temperature for four hours and fifteen minutes then was worked up by quenching with dilute HCl to pH 1 and extracting the product into 20mL toluene. The organic phase was then washed with water and evaporated to 216mg of acid product of Formula **28.**

As shown in Scheme 9, the replacement sidechain is next coupled to the taxane backbone. In the compounds shown in Scheme 9, R₂, R₁₁, R₁₂ and P₃ are as defined above for Scheme 8; R₇, R₈ and R₉ are as defined above for Scheme 7; R₁ is as defined above for Schemes 1 and 2; and R₁₃ and R₁₄ are as defined above for R₈ and R₉, respectively, of Scheme 6. It should be appreciated that the R₁ group in Scheme 9 may be different from the R₁ group that was on the sidechain that was removed in Scheme 7.

Figure 33, for example, provides the coupling reaction of Formula **24** (from Figure 29) with Formula **28** (from Figure 32) to provide the compound of Formula **29** (where R₂=CH₂CH(CH₃)₂; P₃=CBZ; R₁₁=H; R₁₂=PhOMe; R₇=CH₃; R₈=H; R₉=PhOMe in generalized formula **W** of Scheme 9). To a 5mL RBF was charged 180mg (0.255mmol) 7,9-anisaldehyde acetal, 9-desoxo 10-*epi* Baccatin III (Formula **24)** and 105mg (0.510mmol, 2.0 eq.) DCC. Toluene (2mL) was then added to dissolve the solids. Next, 158mg (0.383mmol, 1.5 eq.) *iso*-Butyl sidechain acid (Formula **28)** was dissolved in 1.0mL DCM then this solution was charged to the reaction flask followed by 6mg (0.038mmol, 0.15 eq.) 4-pp. The reaction was stirred at room temperature for 23 hours then was quenched by adding 11.5µL acetic acid and 4µL water and stirring for one hour. MTBE was added to the reaction flask to precipitate DCU and the reaction solution was filtered to remove the precipitate. The filtrate was slurried with activated carbon then passed across a silica plug to remove the 4-Pp salts. The eluent was evaporated to a solid to yield 270.7mg of crude coupled product of Formula **29.**

As exemplified in Figure 34, the 7,9-acetal and N,O-acetal protecting groups may then be removed and an N-acyl group added to form the compounds of Formula **30** and **32** (where R₁=t-butoxyl; R₂=CH₂CH(CH₃)₂; R₇=CH₃ in generalized formula **X** of Scheme 9), which may be separated from each other by liquid chromatography or kept together for the next step. While the same anisaldehyde group is used at both the 7,9-acetal and N,O-acetal in the exemplary compound of Formula 29, such that both groups may be removed in a single step, it should be appreciated that other acetal protecting groups are contemplated such that multiple deprotection steps may be required. To a 10mL RBF was charged, 270mg (0.245mmol) of 7,9-anisaldehyde acetal-10-*epi*-3'-*iso*butyl-3',2'-N,O-anisaldehyde acetal coupled ester of Formula **29,** 220mg (0.8g/g coupled ester) Degussa type palladium on carbon, and 4.1 mL THF. In a separate vial, 99µL conc. HCl was diluted in 198µL water and 1.0mL THF. This solution was added to the reaction flask and the flask was sealed and placed under hydrogen. The hydrogenation reaction was stirred for 31 hours then was quenched by removing the hydrogen and filtering the catalyst from the reaction solution then adding molecular sieves to the reaction solution to remove water before adding 84.5µL (0.368mmol, 1.5 eq.) t-butoxy carbonyl (t-BOC) anhydride then 684µL TEA. The reaction stirred an additional 21 hours then was worked up by filtering the sieves from the reaction solution, diluting the filtrate with EtOAc and washing with water. The organic partition was evaporated to approximately 370mg of oil. The oil was purified first by flash chromatography, then preparative TLC (pTLC) then by a semi-prep reverse phase column to yield 3.9mg of pure product of Formula **30** and **32.**

Finally, as shown in Figure **35,** an alternate 7,9-acetal may be formed if desired to provide the compound of Formula **31** or **33** (where R₁=t-butyl; R₂=CH₂CH(CH₃)₂; R₇=CH₃; R₁₃=H; R₁₄=CH=CH₂ in generalized formula **Y** of Scheme 9). While an acrolein acetal is formed in Figure 35, it should be appreciated that other groups may be substituted for R₁₃ and R₁₄ of Scheme 9, such as those defined for the R₈ and R₉ groups exemplified in Figure 9, or others. In a HPLC vial insert, 3.4mg (4.13µmol) of 9-α-hydroxy, 10-α-acetyl-2'-R,S-hydroxy-3'-S-*iso*butyl-3'-N-t-butoxy carbonyl taxane of Formula **30** and **32** was charged followed by 70µL DCM. Next, 12.8µL of a 1 to 20 diluted acrolein dimethyl acetal in DCM (0.64µL acetal, 5.37µmol, 1.3 eq.) was charged to the insert followed by 8.4µL (0.413µmol, 0.1 eq.) of a 0.05M PTSA solution in DCM. The reaction was lightly agitated then sat at room temperature. The reaction took more additions of the acetal solution to drive it to completion then was worked up after a couple of days by filtering the solution through approximately 80mg of basic activated alumina. The alumina was washed with DCM then EtOAc and the fractions evaporated to dryness. The crude compound was purified on a normal phase analytical column to yield 605µg of compound (the product was an isomeric mixture) 7,9-acrolein acetal-10-α-acetyl-2'-R,S-hydroxy-3'-S-*iso*butyl-3'-N-t-butoxy carbonyl taxane of Formulas **31** and **33,** which may be separated by liquid chromatography.

### VII. Alternative Method for Synthesizing 7,9-Acetal Linked Analogs

7,9 acetal linked analogs of 9,10-αα OH taxanes can also be formed directly from 10-deacetylbaccatin III (10-DAB), which has the formula: 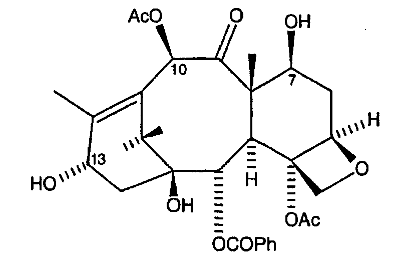 Using 10-DAB has an advantage since it is much more naturally abundant, and thus less expensive than either of the starting compounds **A** or **A'** that are shown and discussed above with respect to in Figures **1** and **2.**

In this alternative process, 10-DAB, Formula **34,** is first protected at both the C-7 and C-10 positions to form C7,C10 di-CBZ 10-deacetylbaccatin III, Formula **35,** according to the following reaction: 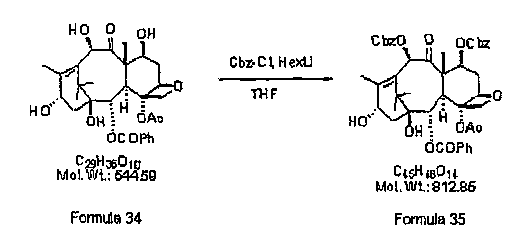 C7, C10 di-CBZ 10-deacetylbaccatin III of Formula **34** (50g, 91.8mmol) was dissolved in THF (2L, 40ml/g) by warming to 40ºC in a warm-water bath. The solution was cooled to -41ºC in a Neslab chiller and benzylchloroformate (46mL, 3.2eq, 293.8 mmol) was added to the stirred chilled solution followed by further cooling to -44ºC. To this solution 2.3M hexyl lithium solution (130mL, 3.3 eq, 303mmol) was added gradually over 45 min while maintaining the temperature of the reaction mixture at ≤ - 39 ºC. Stirring continued in the Neslab for 45 minutes at which time HPLC indicated the reaction had gone to completion. At 2 hr total reaction time, the reaction was quenched by the addition of 1 N HCl (400mL) and IPAc (1L) and removal from the Neslab chiller. The reaction was allowed to stir while warming to 10°C. The layers were separated and the IPAc layer was washed sequentially with H₂O (500mL), saturated NaHCO₃ (200mL) and H₂O (4x500mL) and then filtered through a silica gel pad. The filtrate was concentrated until solids started to form. IPAc (850mL) was added and the mixture was heated to 60 °C to dissolve some of the solids. To the warm solution, heptanes (800mL) were added and the solution was cooled in the refrigerator and filtered. The solids collected by the filtration were washed with heptanes and dried under vacuum at 45ºC to give **35.**

Next, Formula **35** was coupled with a sidechain of Formula **36** to form Formula **37** according to the following reaction: 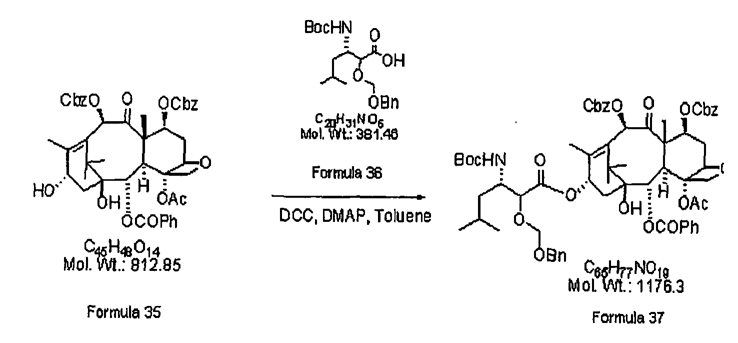 Here, the sidechain of Formula **36,** (38g, 99.6 mmol) was dissolved in toluene to a known concentration (0.09524 g/mL). This solution was added to Formula **35** (54.0g, 66.4mmol). The solution was heated in a warm-water bath and DMAP (8.13g, 66.4 mmol) and DCC (25.28g, 119.6 mmol) in toluene (540mL) were added to the warm reaction mixture. While maintaining the temperature at about 51°C, the reaction was continually stirred and sampled periodically for HPLC. After 3 hours, additional DCC (13.0g) in toluene (140mL) was added.

The following morning (25.25 hr), MTBE (450mL) was added and the reaction mixture was filtered through a pad of silica gel, washed with MTBE followed by EtOAc, and concentrated to give 61.8g oil. The silica was washed again with EtOAc and the second pool was concentrated to 50mL and allowed to sit. The following day the second pool had started to crystallize. It was filtered and the filtrate was washed with 1:1 heptane/IPAc and dried under vacuum at 40ºC to give a solid of Formula **37.**

Next, Formula **37** was deprotected at both the C7 and C10 position to give Formula **38** according to the following reaction: 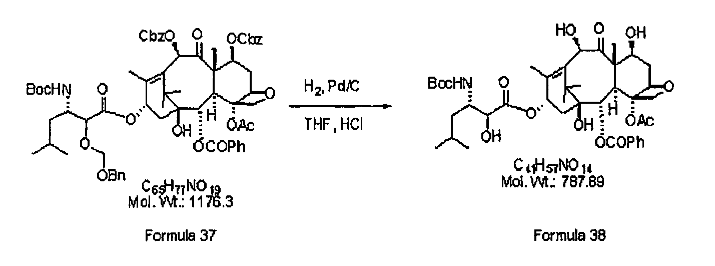 A solution of THF (300 mL) and HCl (22 mL) was added to a solution of Formula **37** (61.8, 52.5mmol) in THF (15 mUg, 920 mL). The resulting solution was flushed with nitrogen. A catalyst (10%Pd/C with 50% water, 99.1g) was added and the flask was flushed with nitrogen three times and then with hydrogen three times. The reaction mixture was stirred vigorously under a hydrogen balloon for 21 hours. At this time the reaction was sampled and HPLC indicated that 38% by area of starting material still remained. Water (10 mL) was added and stirring continued. Twenty hours later, HPLC indicated the same amount of starting material still remaining. The reaction mixture was filtered through celite and washed with THF. It was then concentrated to remove excess THF; fresh catalyst (101 g) was added and the reaction mixture was placed back under hydrogen as before. After another 24 hours, an intermediate compound was still present and still more catalyst (20 g) was added. After another hour, HPLC indicated that the reaction was complete. The reaction mixture was filtered through celite and washed through with IPAc. The combined filtrate was washed with NH₄Cl solution (500mL), water (500mL), 5%NaHCO₃ (500mL), H₂O (300mL), and brine (300mL). The organic layer was dried, filtered, and concentrated to give a foam of Formula **38.**

Formula **38** was then converted to Formula **39** according to the following reaction: 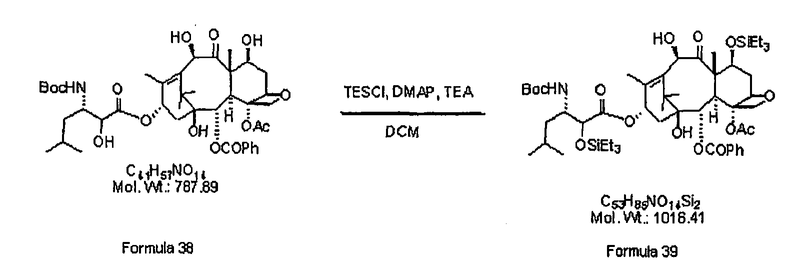 Formula **38** (41.37g, 52.5mmol) was dissolved in DCM (500mL) at room temperature. The solution was cloudy, possibly caused by the presence of DCU in the product from the previous reaction. In the case that the impurity was water, Na₂SO₄ was added to the solution, and the solution was filtered through filter paper into to a 2L flask. The solids were collected and washed with DCM (250mL) into the flask and the flask was covered with a septum and N₂ balloon. Tea (35mL) followed by DMAP (1.284g) and TES-CI (∼30mL, 3.5eq) were added to the solution and stirred. Additional TES-Cl (15mL) and TEA (20mL) were added, and after 6 hours HPLC indicated the reaction had gone to completion.

The reaction was then quenched by the addition of EtOH (25mL). The layers were separated and the organic layer was washed with saturated NH₄Cl (~500mL) and dried over Na₂SO₄ and concentrated. A flash column was packed with silica gel and wet with 8:2 heptane/IPAc (1.5L). The solids were dissolved in 8:2 heptane/IPAc (250mL) and filtered to remove solids that would not dissolve. This solution was concentrated to - 100 mL and applied to the column. The column was eluted with 8:2 heptane/IPAc and fractions collected. Fractions with product were pooled and concentrated to give foam of Formula **39.**

Formula **39** was then oxidized to form Formula **40** according to the following reaction: 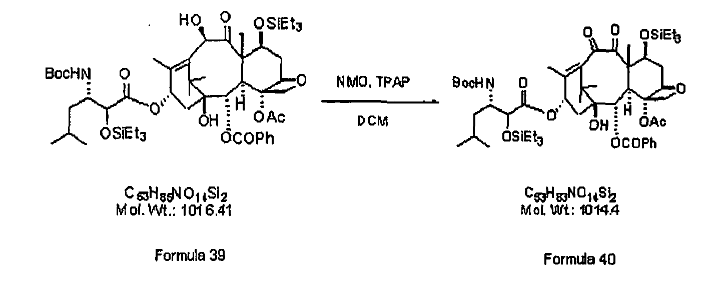 Here, solid Na₂SO₄ was added to a solution of Formula **39** (24.45g, 24.0 mmol) and 4-methyl morpholine N-oxide (10.1g, 84 mmol) in DCM (340mL) to assure that the reaction was dry. The mixture was stirred for 1 hour and then filtered through 24 cm fluted filter paper into a 2L 3-N round bottom flask. The Na₂SO₄ solids were washed with DCM (100mL) into the flask. Molecular sieves (6.1 g, 15wt%/g) were added to the solution and stirring was begun. TPAP (1.38g) was added and the reaction was allowed to stir under a N₂ blanket. Samples were taken periodically for HPLC. Additional TPAP (0.62g) was added after 2 hours and again (0.8g) after 15 hours. The reaction mixture was applied to a pad of silica gel (86g), wet with 8:2 heptane/IPAc and eluted with IPAc. The fractions were collected, pooled and concentrated to an oil. 4-Methyl morpholine N-oxide (5.0g) and DCM (100mL) were added and stirred. Na₂SO₄ (13g) was added to the mixture and it was filtered through filter paper. The Na₂SO₄ solids were washed with DCM (45mL) and molecular sieves (5g) and TPAP (1.03g) were added. After 45 minutes, more TPAP (1.05g) was added. A pad of silica gel was prepared and wet with 80:20 Heptane/IPAc. The reaction mixture was applied to the pad and eluted with IPAc. Fractions were collected and those fractions containing product were pooled and concentrated to give an oil product of Formula **40.**

Next, Formula **40** was reduced according to the following reaction to form Formula **41.**

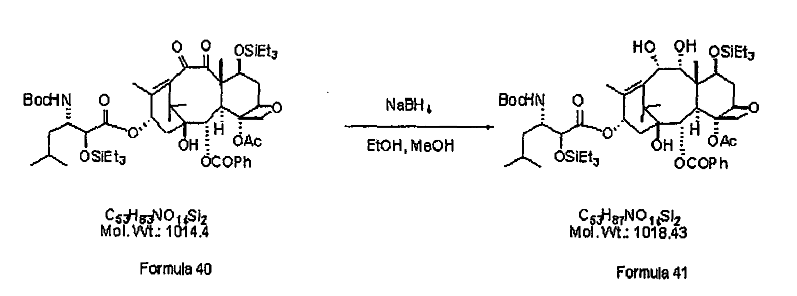

NaBH₄ (365mg, 6 eq) was added to a stirred solution of Formula **40** (1.6g) in EtOH (19mL) and MeOH (6.5mL) cooled in an ice-water bath. After 1 hour, the reaction mixture was removed from the ice-water bath and at 2 hours, the reaction was sampled for HPLC, which indicated the reaction had gone to completion. The reaction mixture was cooled in an ice-water bath and a solution of NH₄OAc in MeOH (15mL) was added followed by the addition of IPAc (50mL) and H₂O (20mL). It was mixed and separated. The organic layer was washed with water (20mL) and brine (10mL), a second time with water (15mL) and brine (10mL), and then twice with water (2x15mL). It was dried over Na₂SO₄ and placed in the freezer overnight. The following morning a sample was taken for HPLC and the reaction was dried and the organic layer was concentrated on the rotovap. It was placed in the vacuum oven to give a foam product of Formula **41.**

Formula **41** was next acylated to form Formula **42** according to the following reaction: 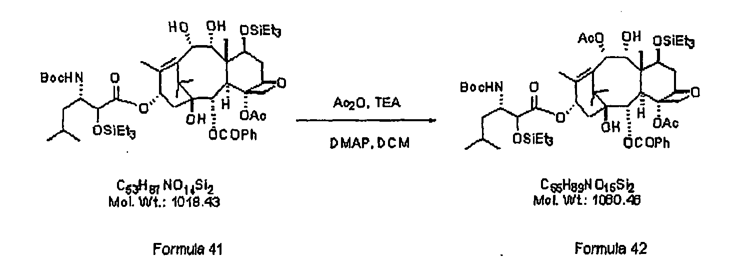 TEA (5.8mL, 41.5mmol), Ac₂O (2.62mL, 27.7mmol) and DMAP (724mg, 5.5mmol) were added to a solution of Formula **41** (14.1g. 13.84mmol)) in DCM (50mL). The reaction was stirred and sampled for HPLC periodically. After 18.5 hours, additional TEA (1.5mL) and Ac₂O (1 mL) were added. At 19 hours, HPLC indicated the reaction had gone to completion. The reaction mixture was diluted with IPAc (300mL) and poured into 5%HaHCO₃ (100ml). It was then stirred, separated, and the organic layer was washed with water (100mL), saturated NH₄Cl (2x100mL), water (3x50mL) and brine (50mL) and then filtered through Na₂SO₄. The mixture was concentrated to give a foam product of Formula **42.**

Next, Formula **42** was converted to a compound of Formula **43** according to the following reaction: 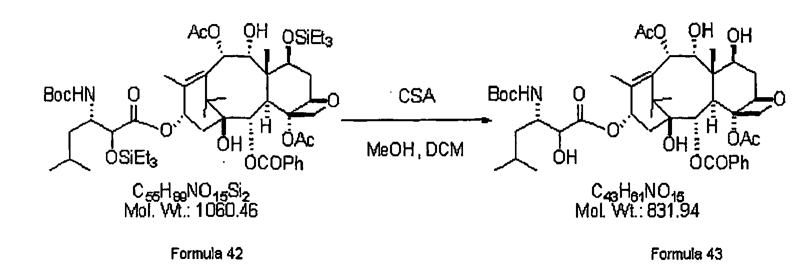 A quantity of Formula **42** (3.0g, 2.829mmol) was weighed into a 100mL flask. Next, DCM (24mL) followed by MeOH (6mL) were added to the flask at room temperature. Stirring of the mixture began under N₂ and CSA (0.0394g, 0.17mmol) was added. After 4 hours LCMS indicated the product had formed. 5%NaHCO₃ (15mL) was added to the reaction mixture; it was shaken vigorously and then added to a separatory funnel. The reaction flask was rinsed into the separatory funnel with 5%NaHCO₃ (25mL) and, thereafter, the reaction mixture was shaken and the layers were separated. The organic layer was washed with brine, dried over Na₂SO₄, and concentrated. MTBE (3x25mL) was added and the reaction mixture was concentrated to dryness after each addition to finally give 3.7068g foam. The foam was dissolved in MTBE (10mL) and stirred. Heptane (50mL) was slowly added to the reaction solution and solids began to form immediately. The solids were vacuum filtered and rinsed with heptane (720mL). The solids were collected and dried in a vacuum oven at 40ºC to give Formula **43.**

Formula **43** was then converted to Formula **44** in the following reaction:

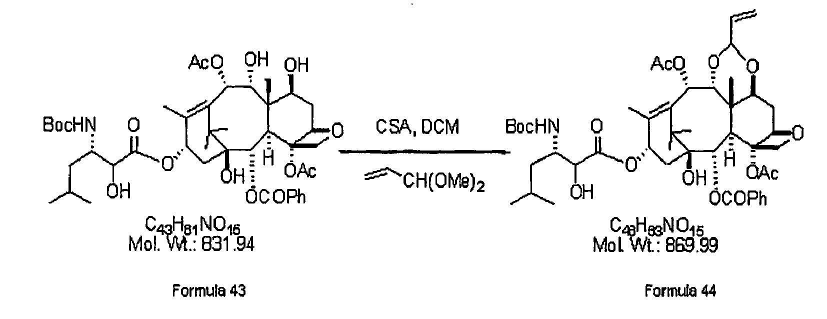

A solution of Formula **43** (2.1 g, 2.52mmol) in DCM (10.5mL) was stirred at room temperature. Next, 3,3-dimethoxy-1-propene (2.03g, 17.7mmol) followed by CSA (0.035g, 0.15mmol) were added to the solution. After the solution was stirred for 3.5 hours, LCMS indicated the reaction had gone to completion. The reaction was diluted with DCM (25mL) and added to a separatory funnel with 55mL 5%NaHCO₃ solution. The layers were separated and the aqueous layer was washed with DCM (25mL). The two organic layers were combined, washed with brine, dried over Na₂SO₄ and concentrated. A flash chromatography column was packed with silica gel and wet with 50:50 MTBE/heptane (1000mL). The reaction mixture was dissolved in MTBE (10mL), loaded on the column and eluted with 50:50 MTBE/heptane. The fractions were collected, pooled, concentrated and dried in a vacuum oven at 50 ºC to give product of Formula **44.**

### IX. Alternate Sidechain Coupling Reaction

As illustrated above in the second reaction step of the alternative process of forming 7,9 acetal linked analogs of 9,10-αα OH taxanes, the C7,C10 di-CBZ 10-deacetylbaccatin III of Formula **35** was coupled with a sidechain of Formula **36** to form Formula **37.** The present invention further contemplates the coupling of an alternative sidechain to Formula **35.** The alternative sidechain of Formula **45** that is contemplated has the following structure: 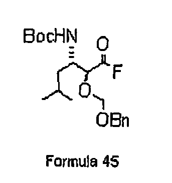 Formula **45** may be formed from the structure of Formula **36** (above) according to the following reaction: 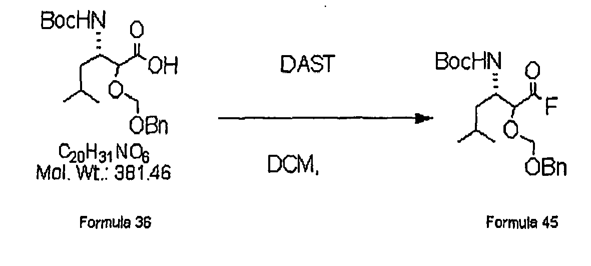 Here, the BOM-acid, Formula **36,** (3.8g, ~10.0mmol) was dissolved in DCM (30mL), stirred and cooled in an ice-water bath at 0 ºC under N₂. DCM (2mL) and diethyl sulfur trifluoride (1.575g, 20.0 mmol) were both added to this solution and the reaction was stirred for 4 hours. The temperature increased to about 10°C. LCMS indicated the reaction had gone to completion. H₂O (50mL) and DCM (50mL) were added and the reaction mixture was transferred to a separatory funnel. The layers were separated and the organic layer was washed with H₂O (50mL) and brine (50mL), dried over Na₂SO₄ and concentrated yielding product of Formula **45.**

Next, Formula **35** was coupled with a sidechain of Formula **45** resulting in product of Formula **46** according to the following reaction: 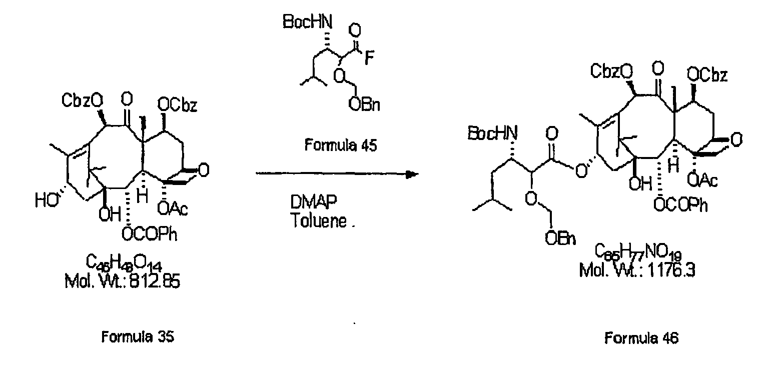 Here, Formula **35** (0.2g, 0.246mmol) and DMAP (0.5g, 4.1 mmol) were weighed into a pear shaped flame-dried flask purged with N₂. An oven-dried reflux condenser, purged with N₂, was placed on top of the flask and it was put in an oil bath heated to 75ºC. The BOM acyl fluoride, Formula **45** (0.5g, 1.31 mmol), in toluene (1 mL) was added to the flask and the temperature increased to 85ºC. Stirring continued under N₂ for 5.5 hour to give product of Formula **46.**

Accordingly, the present invention has been described with some degree of particularity directed to the exemplary embodiments of the present invention. It should be appreciated, though, that the present invention is defined by the following claims construed in light of the prior art so that modifications or changes may be made to the exemplary embodiments of the present invention without departing from the inventive concepts contained herein.

## Claims

1. A compound having the formula: 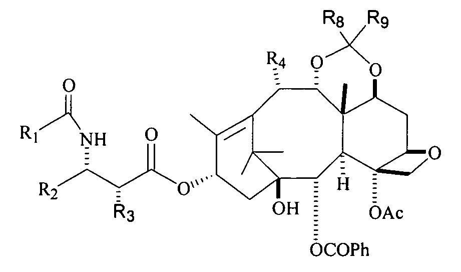 wherein:
R₁ and R₂ are each selected from H, an alkyl group, an olefinic group, an aromatic group, an O-alkyl group, an O-olefinic group, or an O-aromatic group;
R₃ is hydroxyl or OP₁;
R₄ is hydroxyl or R₇COO;
R₇ is an alkyl group, an olefinic group, or an aromatic group;
R₈ and R₉ are each selected from H, an alkyl group, an olefinic group, or an aromatic group; and
P₁ is a hydroxyl protecting group.

2. A chemical compound according to Claim 1 wherein R₄ is R₇COO and wherein R₇COO is selected from the following structures:

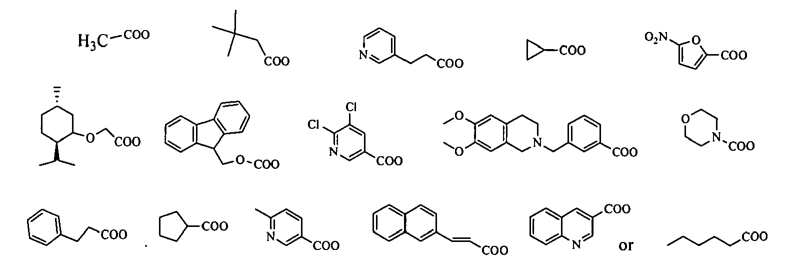

3. A chemical compound according to Claim 1 wherein R₃ is a hydroxyl group, R₈ is H or CH₃, and R₉ is selected from:

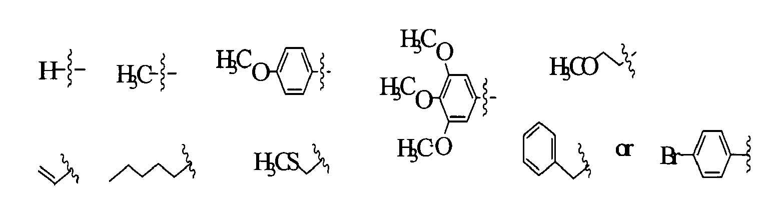

4. A chemical compound according to Claim 1 wherein said compound is selected from formula:-

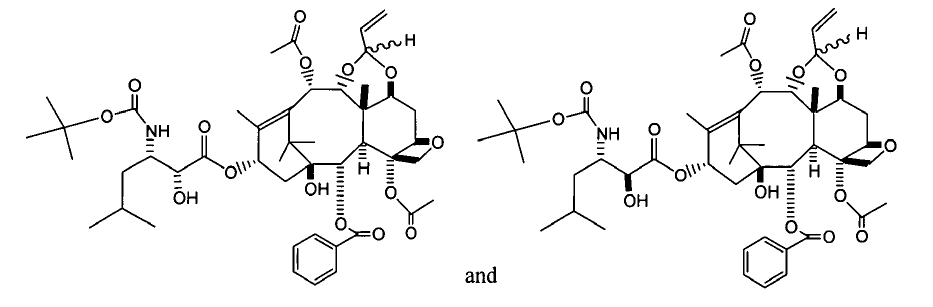

5. A chemical compound according to Claim 1 wherein said compound has the formula 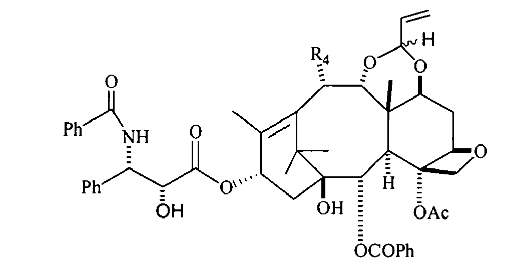 wherein R₄ is hydroxyl or CH₃COO.

6. A chemical compound having the formula: 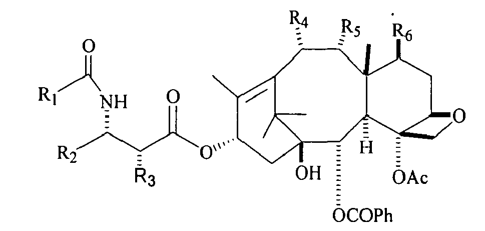 wherein:
R₁ and R₂ are each selected from H, an alkyl group, an olefinic group, an aromatic group, an O-alkyl group, an O-olefinic group, or an O-aromatic group;
R₃ is hydroxyl or OP₁;
R₄ and R₅ are each hydroxyl or R₇COO;
R₆ is hydroxyl, OP₂, R₇COO, or an ether functionality;
R₇ is an alkyl group, an olefinic group, or an aromatic group;
P₁ and P₂ are each hydroxyl protecting groups;
and wherein R₁ and R₂ are not both Ph when
R₃ is OP₁;
R₄ is a hydroxyl;
R₅ is a hydroxyl; and
R₆ is OP₂.

7. A chemical compound according to Claim 6 wherein said compound is selected from the formulae:
